# EUROPEAN PATENT APPLICATION

(11) **EP 3 812 456 A1**
(43) Date of publication of application: **28.04.2021**
(21) Application number: 19793629.7
(22) Date of filing: 22.04.2019
(51) Int. Cl.: C12N 5/077

(54) **GROWTH INHIBITOR**

(30) Priority: 23.04.2018 JP 2018082606
(71) Applicant: Kyoto University, Kyoto-shi, Kyoto 606-8501 (JP); Takeda Pharmaceutical Company Limited, Osaka-shi, Osaka 541-0045 (JP)
(72) Inventor: YAMAZOE, Noriko, Fujisawa-shi, Kanagawa 251-0012 (JP); HIYOSHI, Hideyuki, Fujisawa-shi, Kanagawa 251-0012 (JP); MOCHIDA, Taisuke, Fujisawa-shi, Kanagawa 251-0012 (JP); ITO, Ryo, Fujisawa-shi, Kanagawa 251-0012 (JP); TOYODA, Taro, Kyoto-shi, Kyoto 606-8501 (JP); KIMURA, Azuma, Kyoto-shi, Kyoto 606-8501 (JP)
(74) Representative: Jones, Nicholas Andrew
(86) International application number: PCT/JP2019/017044
(87) International publication number: WO 2019/208505

(57) **Abstract**

The present invention relates to a method for producing an insulin-producing cell population or a pancreatic β cell population having the reduced number of Ki67-positive cells, comprising treating a population of endocrine progenitor cells or cells at a later stage of differentiation with an FGFR1 inhibitor.

## Description

### Technical Field

The present invention relates to a method for removing highly proliferative Ki67-positive cells present in an insulin-producing cell population or a pancreatic β cell population obtained by the induction of differentiation from pluripotent stem cells.

### [Background Art]

Research is underway to induce the differentiation of pluripotent stem cells such as iPS cells or ES cells into insulin-secreting cells such as insulin-producing cells or pancreatic β cells and to apply the obtained cells to the treatment of diabetes mellitus.

Various approaches have been developed and reported so far in order to induce the differentiation of pluripotent stem cells into insulin-secreting cells. Non Patent Literature 1 states that the differentiation of endocrine progenitor cells derived from human iPS cells can be promoted by treating the cells with CAS192705-79-6 (1-[2-amino-6-(3,5-dimethoxyphenyl)-pyrido(2,3-d)pyrimidin-7-yl]-3-tert-butylurea), a fibroblast growth factor receptor (FGFR) 1 inhibitor.

Pyrido(2,3-d)pyrimidine and naphthyridine compounds effective for the inhibition of protein tyrosine kinase are known. The compounds are reportedly useful in the treatment of atherosclerosis, restenosis, and cell proliferative diseases of cancers (Patent Literature 1).

CAS192705-79-6 has further been confirmed to inhibit cell proliferation caused by bFGF stimulation in rat myoblasts but not inhibit cell proliferation caused by PDGE stimulation, and to inhibit the elongation of microvessels in human placental blood vessels, suggesting its applicability as an antiproliferative agent and/or an anti-angiogenic agent targeting tumor growth or new blood vessel formation of atherosclerotic plaques (Non Patent Literature 2).

Meanwhile, it has previously not been known that an insulin-producing cell population or a pancreatic β cell population obtained by further induction of differentiation of a pluripotent stem cell-derived endocrine progenitor cell population or a cell population at a later stage of differentiation includes highly proliferative cells. Furthermore, no discussion has been made on the removal of such cells.

### Citation List

### Patent Literature

Patent Literature 1: WO96/15128

### Non Patent Literature

Non Patent Literature 1: Scientific Reports 6, Article number:35908 (2016)
Non Patent Literature 2: The journal of Pharmacology and Experimental Therapeutics, July 1998, Vol. 286(1), p.569-577

### Summary of Invention

### Technical Problem

The inventors of the present application have found that a cell population obtained by the induction of differentiation of pluripotent stem cells into insulin-producing cells or pancreatic β cells includes highly proliferative cells characterized by being Ki67 marker-positive (hereinafter, referred to as "Ki67-positive cells"), together with these insulin-secreting cells (insulin-producing cells and pancreatic β cells).

In the case of applying insulin-secreting cells obtained by the induction of differentiation to the treatment of diabetes mellitus, etc., it is very important from the viewpoint of safety to strictly control cells other than the insulin-secreting cells. Furthermore, coexisting or remaining highly proliferative cells might adversely affect recipients or influence the long-term graft survival of insulin-secreting cells and are thus not preferred.

Accordingly, an object of the present invention is to provide an approach of removing highly proliferative Ki67-positive cells coexisting with insulin-secreting cells obtained by the induction of differentiation.

### Solution to Problem

The inventors of the present application have conducted diligent studies to attain the object and consequently found that an endocrine progenitor cell population obtained by the induction of differentiation from pluripotent stem cells, or a cell population at a later stage of differentiation is treated with an FGFR1 inhibitor, whereby the proliferation of Ki67-positive cells is inhibited so that an insulin-producing cell population or a pancreatic β cell population having a reduced content of Ki67-positive cells can be obtained.

The present invention is based on these novel findings and encompasses the following inventions.
[1] A method for producing an insulin-producing cell population or a pancreatic β cell population, comprising the step of treating an insulin-producing cell population or a pancreatic β cell population with an FGFR1 inhibitor.
[2] The method according to [1], further comprising the step of differentiating the insulin-producing cell population treated with the FGFR1 inhibitor.
[3] The method according to [1] or [2], wherein the insulin-producing cell population or the pancreatic β cell population is treated with less than 5 µM of the FGFR1 inhibitor.
[4] The method according to any of [1] to [3], wherein the produced cell population comprises Ki67-positive cells at a proportion of less than 3%.
[5] The method according to any of [1] to [4], wherein the FGFR1 inhibitor is 1-[2-amino-6-(3,5-dimethoxyphenyl)-pyrido(2,3-d)pyrimidin-7-yl]-3-tert-butylurea or a salt thereof.
   [5-1] The method according to any of [1] to [4], wherein the FGFR1 inhibitor is a substance having a 50% inhibitory concentration (IC₅₀) of 1 µM or lower against FGFR1.
   [5-2] The method according to any of [1] to [4], wherein the FGFR1 inhibitor is 1-[2-amino-6-(3,5-dimethoxyphenyl)-pyrido(2,3-d)pyrimidin-7-yl]-3-tert-butylurea (CAS No.: 192705-79-6), E-3810 (CAS No.: 1058137-23-7), PD-173074 (CAS No.: 219580-11-7), FGFR4-IN-1 (CAS No.: 1708971-72-5), FGFR-IN-1 (CAS No.: 1448169-71-8), FIIN-2 (CAS No.: 1633044-56-0), AZD4547 (CAS No.: 1035270-39-3), FIIN-3 (CAS No.: 1637735-84-2), NVP-BGJ398 (CAS No.: 1310746-10-1), NVP-BGJ398 (CAS No.: 872511-34-7), CH5183284 (CAS No.: 1265229-25-1), Derazantinib (CAS No.: 1234356-69-4), Derazantinib Racemate, Ferulic acid (CAS No.: 1135-24-6), SSR128129E (CAS No.: 848318-25-2), SSR128129E free acid (CAS No.: 848463-13-8), Erdafitinib (CAS No.: 1346242-81-6), BLU9931 (CAS No.: 1538604-68-0), PRN1371 (CAS No.: 1802929-43-6), S49076 (CAS No.: 1265965-22-7), LY2874455 (CAS No.: 1254473-64-7), Linsitinib (CAS No.: 867160-71-2), Dovitinib (CAS No.: 405169-16-6), Anlotinib (CAS No.: 1058156-90-3), Brivanib (CAS No.: 649735-46-6), Derazantinib (CAS No.: 1234356-69-4), Anlotinib Dihydrochloride (CAS No.: 1360460-82-7), ACTB-1003 (CAS No.: 939805-30-8), BLU-554 (CAS No.: 1707289-21-1), Rogaratinib (CAS No.: 1443530-05-9), BIBF 1120 esylate (CAS No.: 656247-18-6), TG 100572 Hydrochloride (CAS No.: 867331-64-4), ENMD-2076 (CAS No.: 934353-76-1), Brivanib alaninate (CAS No.: 649735-63-7), TG 100572 (CAS No.: 867334-05-2), BIBF 1120 (CAS No.: 656247-17-5), ENMD-2076 Tartrate (CAS No.: 1291074-87-7), TSU-68 (CAS No.: 252916-29-3), Ponatinib (CAS No.: 943319-70-8), Sulfatinib (CAS No.: 1308672-74-3), LY2784544 (CAS No.: 1229236-86-5), Dovitinib lactate (CAS No.: 692737-80-7), SU 5402 (CAS No.: 215543-92-3), FGF-401 (CAS No.: 1708971-55-4), Tyrosine kinase-IN-1 (CAS No.: 705946-27-6), PP58 (CAS No.: 212391-58-7), TG 100801 Hydrochloride (CAS No.: 1018069-81-2), Crenolanib (CAS No.: 670220-88-9), TG 100801 (CAS No.: 867331-82-6), Pazopanib Hydrochloride (CAS No.: 635702-64-6), Pazopanib (CAS No.: 444731-52-6), PD168393 (CAS No.: 194423-15-9), Apatinib (CAS No.: 1218779-75-9), Palbociclib isethionate (CAS No.: 827022-33-3), Foretinib (CAS No.: 849217-64-7), Lenvatinib (CAS No.: 417716-92-8), Tandutinib (CAS No.: 387867-13-2), or a salt thereof.
[6] The method according to any of [2] to [5], wherein the step of differentiating the insulin-producing cell population treated with the FGFR1 inhibitor is performed by transplantation to an animal.
[7] A method for producing an insulin-producing cell population or a pancreatic β cell population, comprising the step of treating a population of endocrine progenitor cells or cells at a later stage of differentiation with less than 5 µM of an FGFR1 inhibitor.
[8] A method for decreasing the number of or inhibiting the proliferation of Ki67-positive cells present in a population of endocrine progenitor cells or cells at a later stage of differentiation, comprising
   treating the cell population with an FGFR1 inhibitor.
   [8-1] A method for inhibiting the proliferation of Ki67-positive cells present in an endocrine progenitor cell population or a cell population at a later stage of differentiation, comprising
      treating the cell population with an FGFR1 inhibitor.
   [8-2] The method according to [8], wherein the absolute number of Ki67-positive cells present in the cell population is decreased.
   [8-3] The method according to any of [8], [8-1], and [8-2], wherein the number of cells that are endocrine progenitor cells or cells at a later stage of differentiation and are not Ki67-positive cells, present in the cell population is not decreased.
[9] The method according to any of [8] to [8-3], wherein the population of endocrine progenitor cells or cells at a later stage of differentiation is an insulin-producing cell population.
[10] The method according to [8] or [9], wherein the population of endocrine progenitor cells or cells at a later stage of differentiation is treated with less than 5 µM of the FGFR1 inhibitor.
[11] The method according to any of [8] to [10], wherein the FGFR1 inhibitor is 1-[2-amino-6-(3,5-dimethoxyphenyl)-pyrido(2,3-d)pyrimidin-7-yl]-3-tert-butylurea or a salt thereof.
[12] A population of pancreatic progenitor cells or cells at a later stage of differentiation, comprising Ki67-positive cells at a proportion of less than 3%.
   [12-1] A population of pancreatic progenitor cells or cells at a later stage of differentiation, comprising Ki67-positive cells at a proportion of less than 2%.
   [12-2] A population of pancreatic progenitor cells or cells at a later stage of differentiation, comprising Ki67-positive cells at a proportion of less than 1%.
   [12A] A population of endocrine progenitor cells or cells at a later stage of differentiation, comprising Ki67-positive cells at a proportion of less than 3%.
   [12A-1] A population of endocrine progenitor cells or cells at a later stage of differentiation, comprising Ki67-positive cells at a proportion of less than 2%.
   [12A-2] A population of endocrine progenitor cells or cells at a later stage of differentiation, comprising Ki67-positive cells at a proportion of less than 1%.
[13] The cell population according to any of [12] to [12-2], wherein the cell population is an insulin-producing cell population.
[14] The cell population according to [12] or [13], wherein the cell population is used for transplantation.
   [14-1] A medicament comprising a cell population according to [12] or [13].
   [14-2] A prodrug comprising a cell population according to [12] or [13].
[15] A method for producing an insulin-producing cell population or a pancreatic β cell population, comprising the steps of:
   (0) including any method described herein (e.g., any method described in the paragraphs [0058] to [0106]);
   (1) treating an insulin-producing cell population or a pancreatic β cell population with an FGFR1 inhibitor; and
   (2) differentiating the insulin-producing cell population treated with the FGFR1 inhibitor.
[16] A method for producing an insulin-producing cell population or a pancreatic β cell population, comprising the steps of:
   (1) treating an insulin-producing cell population or a pancreatic β cell population with an FGFR1 inhibitor; and
   (2) embedding the insulin-producing cell population into a gel containing alginic acid.
[17] A method for producing an insulin-producing cell population or a pancreatic β cell population, comprising the steps of:
   (0) adjusting the purity of a target cell population to at least 70% or more by a method for purifying the target cell population;
   (1) treating an insulin-producing cell population or a pancreatic β cell population with an FGFR1 inhibitor; and
   (2) differentiating the insulin-producing cell population treated with the FGFR1 inhibitor.
[18] A method for treating diabetes mellitus, comprising the step of transplanting a cell population treated with an FGFR1 inhibitor.

The present specification encompasses the contents described in the specification and/or drawings of Japanese Patent Application No. 2018-082606 on which the priority of the present application is based.

All publications, patents and patent applications cited herein are incorporated herein by reference in their entirety.

### Advantageous Effects of Invention

The present invention can provide an approach of removing highly proliferative Ki67-positive cells coexisting with insulin-secreting cells obtained by the induction of differentiation.

### Brief Description of Drawing

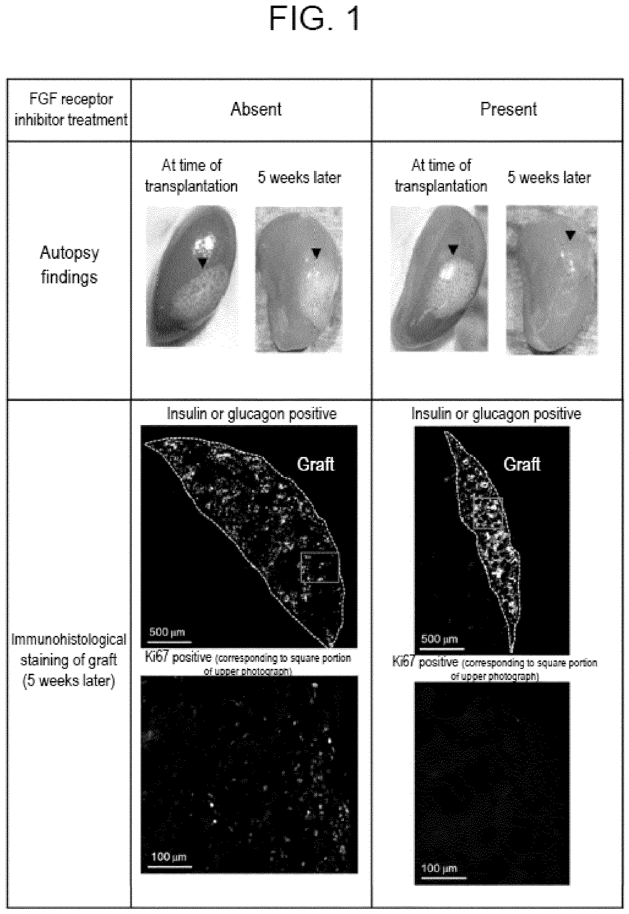

[Figure 1] Figure 1 shows results autopsy findings and immunohistological staining results about a graft excised 5 weeks after transplantation of an insulin-producing cell population obtained by (or without) treatment with an FGFR1 inhibitor. The black arrowhead depicts the graft.

### Description of Embodiments

### 1. Terminology

Hereinafter, the terms described herein will be described.

As used herein, "about" refers to a value which may vary up to plus or minus 25%, 20%, 10%, 8%, 6%, 5%, 4%, 3%, 2%, or 1% from the reference value. Preferably, the term "about" or "around" refers to a range from minus or plus 15%, 10%, 5%, or 1% from the reference value.

As used herein, "comprise(s)" or "comprising" means inclusion of the element(s) following the word without limitation thereto. Accordingly, it indicates inclusion of the element(s) following the word, but does not indicate exclusion of any other element.

As used herein, "consist(s) of" or "consisting of" means inclusion of all the element(s) following the phrase and limitation thereto. Accordingly, the phrase "consist(s) of" or "consisting of" indicates that the enumerated element(s) is required or essential and substantially no other elements exist.

As used herein, "without the use of feeder cell(s)" means basically containing no feeder cells and using no medium preconditioned by culturing feeder cells. Accordingly, the medium does not contain any substance, such as a growth factor or a cytokine, secreted by feeder cells.

"Feeder cells" or "feeder" means cells that are cocultured with another kind of cells, support the cells, and provide an environment that allows the cells to grow. The feeder cells may be derived from the same species as or a different species from the cells that they support. For example, as a feeder for human cells, human skin fibroblasts or human embryonic-stem cells may be used or a primary culture of murine embryonic fibroblasts or immortalized murine embryonic fibroblasts may be used. The feeder cells can be inactivated by exposure to radiation or treatment with mitomycin C.

As used herein, "adhered (adherent)" refers to cells are attached to a container, for example, cells are attached to a cell culture dish or a flask made of a sterilized plastic (or coated plastic) in the presence of an appropriate medium. Some cells cannot be maintained or grow in culture without adhering to the cell culture container. In contrast, non-adherent cells can be maintained and proliferate in culture without adhering to the container.

As used herein, "culture" refers to maintaining, growing, and/or differentiating cells in in vitro environment. "Culturing" means maintaining, proliferating, and/or differentiating cells out of tissue or the living body, for example, in a cell culture dish or flask. The culture includes two-dimensional culture (plane culture) and three-dimensional culture (suspension culture).

As used herein, "enrich(es)" and "enrichment" refer to increasing the amount of a certain component in a composition such as a composition of cells and "enriched" refers, when used to describe a composition of cells, for example, a cell population, to a cell population increased in the amount of a certain component in comparison with the percentage of such component in the cell population before the enrichment. For example, a composition such as a cell population can be enriched for a target cell type and, accordingly, the percentage of the target cell type is increased in comparison with the percentage of the target cells present in the cell population before the enrichment. A cell population can be enriched for a target cell type by a method of selecting and sorting cells known in the art. A cell population can be enriched by a specific process of sorting or selection described herein. In a certain embodiment of the present invention, a cell population is enriched for a target cell population at least 20%, 30%, 40%, 50%, 60%, 70%, 80%, 85%, 90%, 95%, 97%, 98%, or 99% by a method of enriching the target cell population.

As used herein, "deplete(s)" and "depletion" refer to decreasing the amount of a certain component in cells or a composition such as a composition of cells and "depleted" refers, when used to describe cells or a composition of cells, for example, a cell population, to a cell population decreased in the amount of a certain component in comparison with the percentage of such component in the cell population before the depletion. For example, a composition such as a cell population can be depleted for a target cell type and, accordingly, the percentage of the target cell type is decreased in comparison with the percentage of the target cells present in the cell population before the depletion. A cell population can be depleted for a target cell type by a method of selecting and sorting cells known in the art. A cell population can be depleted by a specific process of sorting or selection described herein. In a certain embodiment of the present invention, a cell population is reduced (depleted) for a target cell population at least 50%, 80%, 85%, 90%, 95%, 97%, 98%, or 99% by a method of depleting a target cell population.

As used herein, "purify(ies)" and "purification" refer to removing impurities in a composition such as a composition of cells and making it pure for a certain component and "purified" refers, when used to describe a composition of cells, for example, a cell population, to a cell population in which the amount of impurities is decreased in comparison with the percentage of such components in the cell population before purification and the purity of a certain component is improved. For example, a composition such as a cell population can be purified for a target cell type and, accordingly, the percentage of the target cell type is increased in comparison with the percentage of the target cells present in the cell population before the purification. A cell population can be purified for a target cell type by a method of selecting and sorting cells known in the art. A cell population can be purified by a specific process of sorting or selection described herein. In a certain embodiment of the present invention, the purity of a target cell population is brought by a method of purifying a target cell population to at least 70%, 80%, 85%, 90%, 95%, 97%, 98%, or 99% or to the extent at which impurities (including contaminant cells) are undetectable.

As used herein, "not decrease the number of cells" means that the number of cells is not markedly decreased due to the execution of the method of the present invention and means that there is no marked difference between the number of cells before the execution of the method and the number of cells after the execution of the method. However, decrease in the number of cells that is not caused by the execution of the method of the present invention (e.g., natural death of cells that may usually occur in conventionally known cell culture and differentiation steps) may occur. Thus, "not decrease the number of cells" also includes the case where the rate of decrease in the number of cells after execution of the method of the present invention from that before the execution is 30% or less, 20% or less, 10% or less, or 5% or less.

As used herein, "suppressing proliferation" means that the number of cells is not markedly increased due to the execution of the method of the present invention and means that there is no marked increase between the number of cells before the execution of the method and the number of cells after the execution of the method. "Suppressing proliferation" also includes the case where the rate of increase in the number of cells after execution of the method of the present invention from that before the execution is 30% or less, 20% or less, 10% or less, or 5% or less.

As used herein, "marker" means a cell antigen or a gene thereof that is specifically expressed depending on a predetermined cell type, such as "marker protein" and "marker gene". Preferably, a marker is a cell surface marker and this allows concentration, isolation, and/or detection of living cells. A marker can be a positive selection marker or a negative selection marker.

The detection of a marker protein can be conducted by an immunological assay, for example, ELISA, immunostaining, or flow cytometry using an antibody specific for the marker protein. The detection of a marker gene can be conducted by a method of amplifying and/or detecting nucleic acid known in the art, for example, RT-PCR, microarray, biochip, or the like. As used herein, "positive" for a marker protein means being detected to be positive by flow cytometry and "negative" therefor means being equal to or less than the lower detection limit in flow cytometry. Also, "positive" for a marker gene means being detected by RT-PCR and "negative" therefor means being equal to or less than the lower detection limit in RT-PCR.

As used herein, "expression" is defined as transcription and/or translation of a certain nucleotide sequence driven by an intracellular promoter.

As used herein, "factor having CDK8/19-inhibiting activity" means any substance having the inhibitory activity for CDK8/19. CDK8, in contrast to the other proteins of the same CDK family, is not required for cell proliferation. The inhibition of CDK8 has no great effect under usual conditions. CDK19 and CDK8 are similar to each other as mentioned above. Usually, the inhibition of CDK8 also involves the inhibition of CDK19.

"Growth factors" are endogenous proteins that promote differentiation and/or proliferation of particular cells. Examples of "growth factors" include epidermal growth factor (EGF), acid fibroblast growth factor (aFGF), basic fibroblast growth factor (bFGF), hepatocyte growth factor (HGF), insulin-like growth factor 1 (IGF-1), insulin-like growth factor 2 (IGF-2), keratinocyte growth factor (KGF), nerve growth factor (NGF), platelet-derived growth factor (PDGF), transformation growth factor beta (TGF-β), vascular endothelial growth factor (VEGF), transferrin, various interleukins (for example, IL-1 to IL-18), various colony-stimulating factors (for example, granulocyte/macrophage colony-stimulating factor (GM-CSF)), various interferons (for example, IFN-γ, and the like), and other cytokines having effects on stem cells, for example, stem cell factor (SCF), and erythropoietin (Epo).

As used herein, "ROCK inhibitors" means substances that inhibit Rho kinase (ROCK: Rho-associated, coiled-coil containing protein kinase) and may be substances that inhibit either of ROCK I and ROCK II. The ROCK inhibitors are not particularly limited as long as they have the aforementioned function and examples include N-(4-pyridinyl)-4β-[(R)-1-aminoethyl]cyclohexane-1α-carboxamide (that may be herein also referred to as Y-27632), Fasudil (HA1077), (2S)-2-methyl-1-[(4-methyl-5-isoquinolinyl]sulfonyl]hexahydro-1H-1,4-diazepine (H-1152), 4β-[(1R)-1-aminoethyl]-N-(4-pyridyl)benzene-1zencarboxamide (Wf-536), N-(1H-pyrrolo[2,3-b]pyridin-4-yl)-4PER(R)-1-aminoethyl]cyclohexane-1α-carboxamide (Y-30141), N-(3-{[2-(4-amino-1,2,5-oxadiazol-3-yl)-1-ethyl-1H-imidazo[4,5-c]pyridin-6-yl]oxy}phenyl)-4-{[2-(4-morpholinyl)ethyl]-oxy}benzamide (GSK269962A), N-(6-fluoro-1H-indazol-5-yl)-6-methyl-2-oxo-4-[4-(trifluoromethyl)phenyl]-3,4-dihydro-1H-pyridine-5-carboxamide (GSK429286A). The ROCK inhibitors are not limited to these and antisense oligonucleotides and siRNA to ROCK mRNA, antibodies that bind to ROCK, and dominant negative ROCK mutants can also be used, commercially available, or synthesized according to a known method as ROCK inhibitors.

As used herein, "GSK3β inhibitors" are substances having the inhibitory activity for GSK3β (glycogen synthase kinase 3β). GSK3 (glycogen synthase kinase 3) is a serine/threonine protein kinase and involved in many signaling pathways associated with the production of glycogen, apoptosis, maintenance of stem cells, etc. GSK3 has the 2 isoforms α and β. "GSK3β inhibitors" used in the present invention are not particularly limited as long as they have the GSK3β-inhibiting activity and they may be substances having both the GSK3α-inhibiting activity and the GSK3β-inhibiting activity.

Examples of GSK3β inhibitors include CHIR98014 (2-[[2-[(5-nitro-6-aminopyridin-2-yl)amino]ethyl]amino]-4-(2,4-dichlorophenyl)-5-(1H-imidazol-1-yl)pyrimidine), CHIR99021 (6-[[2-[[4-(2,4-dichlorophenyl)-5-(4-methyl-1H-imidazol-2-yl)-2-pyrimidinyl]amino]ethyl]amino]nicotinonitrile), TDZD-8 (4-benzyl-2-methyl-1,2,4-thiadiazolidine-3,5-dione), SB216763 (3-(2,4-dichlorophenyl)-4-(1-methyl-1H-indol-3-yl)-1H-pyrrole-2,5-dione), TWS-119 (3-[6-(3-aminophenyl)-7H-pyrrolo[2,3-d]pyrimidin-4-yloxy]phenol), kenpaullone, 1-azakenpaullone, SB216763 (3-(2,4-dichlorophenyl)-4-(1-methyl-1H-indol-3-yl)-1H-pyrrole-2,5-dione), SB415286 (3-[(3-chloro-4-hydroxyphenyl)amino]-4-(2-nitrophenyl)-1H-pyrrole-2,5-dione), and AR-AO144-18, CT99021, CT20026, BIO, BIO-acetoxime, pyridocarbazole-ruthenium cyclopentadienyl complex, OTDZT, alpha-4-dibromoacetophenone, lithium, and the like. GSK3β is not limited to these and antisense oligonucleotides and siRNA to GSK3β mRNA, antibodies that bind to GSK3β, dominant negative GSK3β mutants, and the like can also be used, commercially available, or synthesized according to a known method as GSK3β inhibitors.

As used herein, examples of "serum replacement" include Knockout Serum Replacement (KSR: Invitrogen), StemSure Serum Replacement (Wako), B-27 supplement, N2-supplement, albumin (for example, lipid rich albumin), insulin, transferrin, fatty acids, collagen precursors, trace elements (for example, zinc, selenium (for example, sodium selenite)), 2-mercaptoethanol, 3'-thiolglycerol, or mixtures thereof (for example, ITS-G). Preferred serum replacements are B-27 supplement, KSR, StemSure Serum Replacement, ITS-G. The concentration of serum replacement in a medium when added into a medium is 0.01-10% by weight, and preferably 0.1-2% by weight. In the present invention, "serum replacement" is preferably used instead of serum.

### 2. Endocrine progenitor cell population or cell population at later stage of differentiation having suppressed proliferation of Ki67-positive cells

The present invention relates to an endocrine progenitor cell population or a cell population at a later stage of differentiation having suppressed proliferation of Ki67-positive cells. These cell populations can be obtained by treatment with an FGFR1 inhibitor.

As used herein, "Ki67-positive cells" means cells that coexist with endocrine progenitor cells or cells at a later stage of differentiation at least in an endocrine progenitor cell population or a cell population at a later stage of differentiation obtained by the induction of differentiation from pluripotent stem cells in the process of differentiation of the pluripotent stem cells into pancreatic β cells, and are characterized by the expression of Ki67 as a marker.

"Ki67" is known as a cell cycle-related nucleoprotein and is also known as a marker for cell proliferation and cell cycle because its expression is found in the G1, S, G2, and M phases of proliferating cells and is not found in the G0 phase, a quiescent stage.

It is known that cells having different features depending on the stages of differentiation appear in the process of differentiation of pluripotent stem cells into pancreatic β cells (WO2009/012428 and WO2016/021734). For example, the stages of differentiation can be broadly classified into pluripotent stem cells, definitive endoderm cells, primitive gut tube cells, posterior foregut cells, pancreatic progenitor cells, endocrine progenitor cells, insulin-producing cells, and pancreatic β cells in order from relatively undifferentiated to differentiated forms.

As used herein, "pluripotency" means the ability to differentiate into tissues and cells having various different shapes and functions and to differentiate into cells of any lineage of the 3 germ layers. "Pluripotency" is different from "totipotency", which is the ability to differentiate into any tissue of the living body, including the placenta, in that pluripotent cells cannot differentiate into the placenta and therefore, do not have the ability to form an individual.

As used herein, "multipotency" means the ability to differentiate into plural and limited numbers of linages of cells. For example, mesenchymal stem cells, hematopoietic stem cells, neural stem cells are multipotent, but not pluripotent.

As used herein, "pluripotent stem cells" refers to embryonic-stem cells (ES cells) and cells potentially having a pluripotency similar to that of ES cells, that is, the ability to differentiate into various tissues (all of the endodermal, mesodermal, and ectodermal tissues) in the living body. Examples of cells having a pluripotency similar to that of ES cells include "induced pluripotent stem cells" (that may be herein also referred to as "iPS cells"). In the present invention, preferably, pluripotent stem cells are human pluripotent stem cells.

Available "ES cells" include murine ES cells, such as various murine ES cell lines established by inGenious, RIKEN, and the like, and human ES cells, such as various human ES cell lines established by NIH, RIKEN, Kyoto University, Cellartis, and the like. For example, available ES cell lines include CHB-1 to CHB-12, RUES1, RUES2, HUES1 to HUES28 from NIH, and the like; H1 and H9 from WisCell Research; and KhES-1, KhES-2, KhES-3, KhES-4, KhES-5, SSES1, SSES2, SSES3 from RIKEN, and the like.

"Induced pluripotent stem cells" refers to cells that are obtained by reprograming mammalian somatic cells or undifferentiated stem cells by introducing particular factors (nuclear reprogramming factors). At present, there are various "induced pluripotent stem cells" and iPS cells established by Yamanaka, et al. by introducing the 4 factors Oct3/4, Sox2, Klf4, c-Myc into murine fibroblasts (Takahashi K, Yamanaka S., Cell, (2006) 126: 663-676); iPS cells derived from human cells, established by introducing similar 4 factors into human fibroblasts (Takahashi K, Yamanaka S., et al. Cell, (2007) 131: 861-872.); Nanog-iPS cells established by sorting cells using expression of Nanog as an indicator after introduction of the 4 factors (Okita, K., Ichisaka, T., and Yamanaka, S. (2007). Nature 448, 313-317.); iPS cells produced by a method not using c-Myc (Nakagawa M, Yamanaka S., et al. Nature Biotechnology, (2008) 26, 101-106); and iPS cells established by introducing 6 factors in a virus-free way (Okita K et al. Nat. Methods 2011 May; 8(5): 409-12, Okita K et al. Stem Cells. 31 (3) 458-66) may be also used. Also, induced pluripotent stem cells established by introducing the 4 factors OCT3/4, SOX2, NANOG, and LIN28 by Thomson et al. (Yu J., Thomson JA. et al., Science (2007) 318: 1917-1920.); induced pluripotent stem cells produced by Daley et al. (Park IH, Daley GQ.et al., Nature (2007) 451: 141-146); induced pluripotent stem cells produced by Sakurada et al. (Japanese Unexamined Patent Application Publication No. 2008-307007) and the like may be used.

In addition, any of known induced pluripotent stem cells known in the art described in all published articles (for example, Shi Y., Ding S., et al., Cell Stem Cell, (2008) Vol 3, Issue 5, 568-574; Kim JB., Scholer HR., et al., Nature, (2008) 454, 646-650; Huangfu D., Melton, DA., et al., Nature Biotechnology, (2008) 26, No. 7, 795-797) or patents (for example, Japanese Unexamined Patent Application Publication No. 2008-307007, Japanese Unexamined Patent Application Publication No. 2008-283972, US2008-2336610, US2009-047263, WO2007-069666, WO2008-118220, WO2008-124133, WO2008-151058, WO2009-006930, WO2009-006997, WO2009-007852) may be used.

Available induced pluripotent cell lines include various iPS cell lines established by NIH, Institute of Physical and Chemical Research (RIKEN), Kyoto University and the like. For example, such human iPS cell lines include the RIKEN cell lines HiPS-RIKEN-1A, HiPS-RIKEN-2A, HiPS-RIKEN-12A, and Nips-B2 and the Kyoto University cell lines Ff-WJ-18, Ff-I01s01, Ff-I01s02, Ff-I01s04, Ff-I01s06, Ff-I14s03, Ff-I14s04, QHJI01s01, QHJI01s04, QHJI14s03, QHJI14s04, 253G1, 201B7, 409B2, 454E2, 606A1, 610B1, 648A1, CDI cell lines MyCell iPS Cells (21525.102.10A), MyCell iPS Cells (21526.101.10A), and the like.

As used herein, "pancreatic progenitor cell population" means a cell population characterized by pancreatic progenitor cells. As used herein, pancreatic progenitor cells mean cells characterized by the expression of at least one of the markers PDX-1, NKX6-1, PTF-1α, GATA4 and SOX9.

The pancreatic progenitor cell population is a cell population comprising pancreatic progenitor cells at a proportion of 30% or more, preferably 50% or more, more preferably 70% or more. The pancreatic progenitor cell population may include other cells (for example, endocrine progenitor cells, insulin-producing cells, and Ki67-positive cells), in addition to the pancreatic progenitor cells.

As used herein, "endocrine progenitor cell population" means a cell population characterized by endocrine progenitor cells.

As used herein, endocrine progenitor cells mean cells characterized by the expression of at least one of the markers Chromogranin A, NeuroD and NGN3 and no expression of a marker of the pancreas-related hormone system (for example, insulin). The endocrine progenitor cells may express a marker such as PAX-4, NKX2-2, Islet-1, PDX-1, or PTF-1α.

The endocrine progenitor cell population is a cell population comprising endocrine progenitor cells at a proportion of 30% or more, preferably 50% or more, more preferably 70% or more. The endocrine progenitor cell population may include other cells (for example, pancreatic progenitor cells, insulin-producing cells, and Ki67-positive cells), in addition to the endocrine progenitor cells.

The proportion of specific cells in a cell population can be determined on the basis of a known approach capable of calculating the number of cells, such as flow cytometry.

"Cell population at a later stage of differentiation" means a cell population characterized by cells whose stage of differentiation into pancreatic β cells are more advanced than that of endocrine progenitor cells. The cells whose stage of differentiation into pancreatic β cells are more advanced than that of endocrine progenitor cells are preferably insulin-producing cells or pancreatic β cells.

As used herein, "insulin-producing cells" means cells characterized in that the expression of a marker of insulin is found and the expression level of NGN3 is at a proportion of less than 1/3 of the maximum expression confirmed in endocrine progenitor cells. "Insulin-producing cells" are cells that may express a marker of NKX6.1 and preferably express both markers of insulin and NKX6.1 and have a NGN3 expression level at a proportion of less than 1/3 of the maximum expression confirmed in endocrine progenitor cells.

The NGN3 expression level can be measured by quantitative RT-PCR. cDNA prepared from cells at a predetermined stage using Cells-to-CT kit (Thermo Fisher Scientific) is used in measurement using TaqMan probe/primer sequence sets against NGN3 and GAPDH, and then, a relative expression level to the GAPDH expression level is regarded as the gene expression level of NGN3.

"Insulin-producing cell population" is a cell population comprising insulin-producing cells at a proportion of 5% or more, preferably 15% or more, more preferably 30% or more. The cell population may include other cells (for example, endocrine progenitor cells; other pancreatic hormone-producing cells expressing at least one of the markers glucagon, somatostatin, and pancreatic polypeptide; and Ki67-positive cells), in addition to the insulin-producing cells.

The insulin-producing cell population can be further divided into "early insulin-producing cell population" which appears at an early stage of a differentiation process from endocrine progenitor cells and "late insulin-producing cell population" which appears at a more advanced stage of differentiation. The late insulin-producing cell population can be characterized, particularly, by including insulin-positive/NKX6.1-positive cells (INS/NKX6.1 double-positive cells) at a proportion of 20% or more.

As used herein, "pancreatic β cells" means cells more mature than "insulin-producing cells" and specifically means cells characterized by expressing at least one of the markers MAFA, UCN3, and IAPP, which are maturation markers of pancreatic β cells, or by a reaction to increase insulin secretion by glucose stimulation.

"Pancreatic β cell population" is a cell population comprising pancreatic β cells that can be obtained by the differentiation and/or maturation, preferably the *in vivo* differentiation and/or maturation, of an endocrine progenitor cell population or a cell population at a later stage of differentiation. The cell population may include other cells (for example, insulin-producing cells and Ki67-positive cells), in addition to the pancreatic β cells.

The endocrine progenitor cell population or the cell population at a later stage of differentiation can be obtained by use of a known approach of inducing the differentiation of pluripotent stem cells into pancreatic β cells. Specifically, each cell population of interest can be obtained using the following steps of induction of differentiation:
step 1) inducing the differentiation of pluripotent stem cells into definitive endoderm cells;
step 2) inducing the differentiation of the definitive endoderm cells into primitive gut tube cells;
step 3) inducing the differentiation of the primitive gut tube cells into posterior foregut cells;
step 4) inducing the differentiation of the posterior foregut cells into pancreatic progenitor cells;
step 5) inducing the differentiation of the pancreatic progenitor cells into endocrine progenitor cells; and
step 6) inducing the differentiation of the endocrine progenitor cells into insulin-producing cells.

Hereinafter, each step will be described, though the induction of differentiation into each cell is not limited by these approaches.

### Step 1) Differentiation into definitive endoderm cells

The pluripotent stem cells are first allowed to differentiate into definitive endoderm cells. Methods for inducing the definitive endoderm from pluripotent stem cells have already been known, and any of the methods may be used. Preferably, the pluripotent stem cells are cultured in a medium containing activin A, more preferably a medium containing activin A, a ROCK inhibitor, and a GSK3β inhibitor, to thereby differentiate into definitive endoderm cells. The number of cells at the start of culture is not particularly limited and is 22000 to 150000 cells/cm², preferably 22000 to 100000 cells/cm², more preferably 22000 to 80000 cells/cm². The culture period is 1 day to 4 days, preferably 1 day to 3 days, particularly preferably 3 days.

The culture temperature is not particularly limited, and the culture is performed at 30 to 40°C (for example, 37°C). The concentration of carbon dioxide in a culture container is on the order of, for example, 5%.

The medium used in this step may be a basal medium for use in the culture of mammalian cells, such as RPMI 1640 medium, MEM medium, iMEM medium, DMEM/F12 medium, Improved MEM Zinc Option medium, Improved MEM/1% B27/Penicillin Streptomycin medium, or MCDB131/20 mM Glucose/NaHCO₃/FAF-BSA/ITS-X/Glutamax/ascorbic acid/Penicillin Streptomycin medium.

The concentration of the activin A in the medium is usually 30 to 200 ng/mL, preferably 50 to 150 ng/mL, more preferably 70 to 120 ng/mL, particularly preferably about 100 ng/mL.

In another embodiment, the activin A can be contained at a low dose, for example, in an amount of 5 to 100 ng/mL, preferably 5 to 50 ng/mL, more preferably 5 to 10 ng/mL, in the medium.

In an alternative embodiment, the concentration of the activin A in the medium is about 0.1 to 100 ng/ml, preferably about 1 to 50 ng/ml, more preferably about 3 to 10 ng/ml.

The concentration of the GSK3β inhibitor in the medium is appropriately set depending on the type of the GSK3β inhibitor used. For example, in the case of using CHIR99021 as the GSK3β inhibitor, its concentration is usually 2 to 5 µM, preferably 2 to 4 µM, particularly preferably about 3 µM.

The concentration of the ROCK inhibitor in the medium is appropriately set depending on the type of the ROCK inhibitor used. For example, in the case of using Y27632 as the ROCK inhibitor, its concentration is usually 5 to 20 µM, preferably 5 to 15 µM, particularly preferably about 10 µM.

The medium can be further supplemented with insulin. The insulin can be contained in an amount of 0.01 to 20 µM, preferably 0.1 to 10 µM, more preferably 0.5 to 5 µM, in the medium. The concentration of the insulin in the medium may be, but is not limited to, the concentration of insulin contained in added B-27 supplement.

Specifically, the cells are cultured for 1 day in a medium containing activin A, a ROCK inhibitor, and a GSK3β inhibitor and then further cultured for 2 days in a medium containing only activin A with the medium replaced with a fresh one every day. Alternatively, the pluripotent stem cells can be subjected to first culture in a medium containing 0.01 to 20 µM insulin in the presence of a low dose of activin A and subsequently subjected to second culture in an insulin-free medium, for production.

### Step 2) Differentiation into primitive gut tube cells

The definitive endoderm cells obtained in step 1) are further cultured in a medium containing a growth factor to induce their differentiation into primitive gut tube cells. The culture period is 2 days to 8 days, preferably about 4 days.

The culture temperature is not particularly limited, and the culture is performed at 30 to 40°C (for example, 37°C). The concentration of carbon dioxide in a culture container is on the order of, for example, 5%. The culture may be performed by any of two-dimensional culture and three-dimensional culture.

A basal medium for use in the culture of mammalian cells can be used as culture medium, as in step 1). The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM). For example, in the case of using KGF as the growth factor, its concentration is usually 5 to 150 ng/mL, preferably 30 to 100 ng/mL, particularly preferably about 50 ng/mL.

### Step 3) Differentiation into posterior foregut cells

The primitive gut tube cells obtained in step 2) are further cultured in a medium containing a growth factor, cyclopamine, noggin, and the like to induce their differentiation into posterior foregut cells. The culture period is 1 day to 5 days, preferably about 2 days. The culture may be performed by any of two-dimensional culture and three-dimensional culture.

The culture temperature is not particularly limited, and the culture is performed at 30 to 40°C (for example, 37°C). The concentration of carbon dioxide in a culture container is on the order of, for example, 5%.

As in step 1), a basal medium for use in the culture of mammalian cells can be used as culture medium. The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM). For example, in the case of using KGF as the growth factor, its concentration is usually 5 to 150 ng/mL, preferably 30 to 100 ng/mL, particularly preferably about 50 ng/mL.

The concentration of the cyclopamine in the medium is not particularly limited and is usually 0.5 to 1.5 µM, preferably 0.3 to 1.0 µM, particularly preferably about 0.5 µM.

The concentration of the noggin in the medium is not particularly limited and is usually 10 to 200 ng/mL, preferably 50 to 150 ng/mL, particularly preferably about 100 ng/mL.

### Step 4) Differentiation into pancreatic progenitor cells

The posterior foregut cells obtained in step 3) are further cultured in a medium containing a factor having CDK8/19-inhibiting activity, preferably a medium containing a factor having CDK8/19-inhibiting activity and a growth factor, to induce their differentiation into pancreatic progenitor cells. The culture period is 2 days to 10 days, preferably about 5 days. The culture may be performed by any of two-dimensional culture and three-dimensional culture.

In the case of two-dimensional culture, according to the previous report (Toyoda et al., Stem cell Research (2015) 14, 185-197), the posterior foregut cells obtained in step 3) are treated and dispersed by pipetting with 0.25% trypsin-EDTA and the dispersion is subjected to centrifugal separation to obtain cell suspension and then the suspension is reseeded to a fresh medium of step 4.

As in step 1), a basal medium for use in the culture of mammalian cells can be used as culture medium. The medium may be appropriately supplemented with a serum replacement, a vitamin, an antibiotic, and the like, in addition to the growth factor.

Each of the compounds mentioned above or salts thereof can be used as the factor having CDK8/19-inhibiting activity. The amount of the factor added to the medium is appropriately determined according to the compound or the salt thereof used and is usually about 0.00001 µM to 5 µM, preferably 0.00001 µM to 1 µM. The concentration of the factor having CDK8/19-inhibiting activity in the medium is preferably a concentration that attains inhibitory activity of 50% or more for CDK8/19.

The growth factor is preferably EGF, KGF, and/or FGF10, more preferably KGF and/or EGF, further preferably KGF and EGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM). For example, in the case of using KGF and EGF as the growth factor, the concentration of EGF is usually 5 to 150 ng/mL, preferably 30 to 100 ng/mL, particularly preferably about 50 ng/mL, and the concentration of KGF is usually 10 to 200 ng/mL, preferably 50 to 150 ng/mL, particularly preferably about 100 ng/mL.

Culture on the first day in step 4) may be performed in the presence of a ROCK inhibitor, and culture on the following days may be performed in a medium containing no ROCK inhibitor.

The medium may also contain a PKC activator. PdBU (PKC activator II), TPB (PKC activator V), or the like is used as the PKC activator, though the PKC activator is not limited thereto. The concentration of the PKC activator to be added is about 0.1 to 100 ng/ml, preferably about 1 to 50 ng/ml, more preferably about 3 to 10 ng/ml.

The medium may also be supplemented with dimethyl sulfoxide and/or activin (1 to 50 ng/ml).

In any of the steps, the medium may be supplemented with a serum replacement (for example, B-27 supplement, ITS-G), in addition to the components described above. Also, an amino acid, L-glutamine, GlutaMAX (product name), a non-essential amino acid, a vitamin, nicotinamide, an antibiotic (for example, Antibiotic-Antimycotic (also referred to as AA herein), penicillin, streptomycin, or a mixture thereof), an antimicrobial agent (for example, amphotericin B), an antioxidant, pyruvic acid, a buffer, inorganic salts, and the like may be added thereto, if necessary. In the case of adding an antibiotic to the medium, its concentration in the medium is usually 0.01 to 20% by weight, preferably 0.1 to 10% by weight. The culture may be performed by any of two-dimensional culture and three-dimensional culture.

In the case of two-dimensional culture, the cell culture is performed by adherent culture without the use of feeder cells. For the culture, a culture container, for example, a dish, a flask, a microplate, or a cell culture sheet such as OptiCell (product name) (Nunc), is used. The culture container is preferably surface-treated in order to improve adhesiveness to cells (hydrophilicity), or coated with a substrate for cell adhesion such as collagen, gelatin, poly-L-lysine, poly-D-lysine, laminin, fibronectin, Matrigel (for example, BD Matrigel (Nippon Becton Dickinson Company, Ltd.)), or vitronectin. The culture container is preferably a culture container coated with type I-collagen, Matrigel, fibronectin, vitronectin or poly-D-lysine, more preferably a culture container coated with Matrigel or poly-D-lysine.

The culture temperature is not particularly limited, and the culture is performed at 30 to 40°C (for example, 37°C). The concentration of carbon dioxide in a culture container is on the order of, for example, 5%.

The pancreatic progenitor cells obtained in step 4) can be further purified using a known surface marker glycoprotein 2 (GP2) or the like. The purification can be performed by a method known per se, for example, using anti-GP2 antibody-immobilized beads.

### Step 5) Differentiation into endocrine progenitor cells

The pancreatic progenitor cells obtained in step 4) are further cultured in a medium containing a growth factor to induce their differentiation into endocrine progenitor cells. The culture may be performed by any of two-dimensional culture and three-dimensional culture. In the case of two-dimensional culture, the pancreatic progenitor cells obtained in step 4) are treated and dispersed by pipetting with 0.25% trypsin-EDTA and the dispersion is subjected to centrifugal separation to obtain cell suspension and then the suspension is reseeded to a fresh medium of step 5). The culture period is 2 days to 3 days, preferably about 2 days.

As in step 1), a basal medium for use in the culture of mammalian cells can be used as culture medium. The medium is supplemented with SANT1, retinoic acid, ALK5 inhibitor II, T3, and LDN according to the previous report (Nature Biotechnology 2014; 32: 1121-1133) and may be appropriately further supplemented with a Wnt inhibitor, a ROCK inhibitor, FGF (preferably FGF2), a serum replacement, a vitamin, an antibiotic, and the like.

The culture is performed by nonadherent culture without the use of feeder cells. For the culture, a dish, a flask, a microplate, a porous plate (Nunc), or the like, or a bioreactor is used. The culture container is preferably surface-treated in order to decrease adhesiveness to cells.

The culture temperature is not particularly limited, and the culture is performed at 30 to 40°C (for example, 37°C). The concentration of carbon dioxide in a culture container is on the order of, for example, 5%.

### Step 6) Differentiation into insulin-producing cells

The endocrine progenitor cells obtained in step 5) are further cultured in a medium containing a growth factor to induce their differentiation into insulin-producing cells. The culture period is 10 days to 30 days, preferably about 10 to 20 days.

As in step 1), a basal medium for use in the culture of mammalian cells can be used as culture medium. The medium is supplemented with ALK5 inhibitor II, T3, LDN, γ-secretase inhibitor XX, γ-secretase inhibitor RO, N-cysteine, an AXL inhibitor, and ascorbic acid according to the previous report (Nature Biotechnology 2014; 32: 1121-1133) and may be appropriately further supplemented with a Wnt inhibitor, a ROCK inhibitor, FGF (preferably FGF2), a serum replacement, a vitamin, an antibiotic, and the like. For example, the medium may be supplemented with ALK5 inhibitor II, T3, LDN, γ-secretase inhibitor RO, and ascorbic acid or may be supplemented with T3, ALK5 inhibitor II, ZnSO₄, heparin, N-acetylcysteine, Trolox, and R428.

The culture may be performed by any of two-dimensional culture and three-dimensional culture. The culture is performed by nonadherent culture without the use of feeder cells. For the culture, a dish, a flask, a microplate, a porous plate (Nunc), or the like, or a bioreactor is used. The culture container is preferably surface-treated in order to decrease adhesiveness to cells.

The culture temperature is not particularly limited, and the culture is performed at 30 to 40°C (for example, 37°C). The concentration of carbon dioxide in a culture container is on the order of, for example, 5%.

### Differentiation into pancreatic β cells

The cells obtained in the preceding step can be induced to differentiate into pancreatic β cells. The step of differentiation into a pancreatic β cell population can be performed by transplanting an endocrine progenitor cell population or a cell population at a later stage of differentiation, preferably an insulin-producing cell population, into a living body of an animal.

"Animal" is preferably a mammal. Examples thereof include humans, nonhuman primates, pigs, cattle, horses, sheep, goats, llamas, dogs, cats, rabbits, mice, and guinea pigs. A human is preferred.

The transplantation is preferably performed to an *in vivo* region where the cell population can be fixed at a given position, and can be performed, for example, subcutaneously, intraperitoneally, to the peritoneal mesothelium, to the greater omentum, to a fat tissue, to a muscle tissue, or beneath the capsule of each organ such as the pancreas or the kidney, in the animal. The number of cells to be transplanted may vary depending on factors such as the stage of differentiation of the cells to be transplanted, the age and body weight of a recipient, the size of a transplantation site, and the severity of a disease and is not particularly limited. For example, the number of cells can be on the order of 10 × 10⁴ cells to 10 × 10¹¹ cells. The transplanted cell population is induced to differentiate in an *in vivo* environment and can thereby differentiate into the cell population of interest, preferably a pancreatic β cell population, which may then be recovered or may be indwelled *in vivo* as it is.

For the transplantation, the cell population may be embedded in a gel containing alginic acid and then transplanted. For example, the cell population embedded in the gel containing alginic acid may be enclosed in a device such as a capsule, a bag, or a chamber and transplanted into a living body.

"Gel containing alginic acid" can be prepared in accordance with a known approach (WO2010/032242 and WO2011/154941) and can be obtained by adding a cross-linking agent to a solution of alginate or alginic acid ester for gelation.

The alginate can be a water-soluble salt, and a metal salt, an ammonium salt, or the like can be used. For example, sodium alginate, calcium alginate, or ammonium alginate can be suitably used.

The alginic acid ester (also referred to as propylene glycol alginate) is a derivative in which propylene glycol is bonded to the carboxyl group of alginic acid through an ester bond.

The ratio of mannuronic acid to guluronic acid (M/G ratio) contained in the alginate is arbitrary. In general, in the case of M > G, a highly flexible gel can be formed. In the case of M < G, a strong gel can be formed. In the present invention, alginate containing guluronic acid at a proportion of 10 to 90%, 20 to 80%, 30 to 70%, or 40 to 60% can be used.

The alginate or the alginic acid ester can be contained in an amount of 0.05 to 10% by weight, preferably 0.1 to 5% by weight, more preferably 0.5 to 3% by weight, in a solvent. The solvent can be any solvent capable of dissolving the alginate or the alginic acid ester, and water, physiological saline, or the like can be used.

The cross-linking agent can be any cross-linking agent that can allow a solution of alginate or alginic acid ester to gelate, and is not particularly limited. A polyvalent metal cation can be used. The polyvalent metal cation is preferably a divalent metal cation, more preferably a calcium ion, a strontium ion, or a barium ion. The cross-linking agent can be used in the form of a salt. In the present invention, at least one member selected from calcium chloride, strontium chloride, and barium chloride can be used as the cross-linking agent.

The gel containing alginic acid can contain a nanofiber. The nanofiber is a natural or synthetic fiber having a diameter of a nanometer order. Examples of the natural nanofiber include nanofibers containing one or more of collagen, cellulose, silk fibroin, keratin, gelatin, and polysaccharides such as chitosan. Examples of the synthetic nanofiber include polylactic acid (PLA), polycaprolactone (PCL), polyurethane (PU), poly(lactide-co-glycolide) (PLGA), poly(3-hydroxybutyrate-co-hydroxyvalerate) (PHBV), and poly(ethylene-co-vinylacetate) (PEVA). The nanofiber can be contained in an amount of less than 1% by weight, for example, 0.9% by weight, 0.8% by weight, 0.7% by weight, 0.6% by weight, 0.5% by weight, or less than the amount, in the gel containing alginic acid. The lower limit of the amount of the nanofiber contained in the gel containing alginic acid is not particularly limited and can be 0.05% by weight or more, preferably 0.1% by weight or more.

In the present invention, "embedding" means that an endocrine progenitor cell population or a cell population at a later stage of differentiation is contained in a scattered manner in the gel containing alginic acid.

The embedding of the cell population in the gel containing alginic acid can be performed by mixing the cell population into a solution of alginate or alginic acid ester and gelating it.

The cell population can be contained in an amount selected from 1 × 10⁴ cells to 1 × 10⁹ cells/mL, preferably 1 × 10⁷ cells to 1 × 10⁸ cells/mL, in the solution of alginate or alginic acid ester.

The gelation of the solution of alginate or alginic acid ester containing the cell population can be performed by adding a cross-linking agent to the solution. The amount of the cross-linking agent added can be an amount selected from 0.1 to 5% by weight, for example, 0.1 to 1% by weight, with respect to the solution. The gelation can be performed in a container having a predetermined configuration and/or shape for use in cell culture or cell transplantation, or in a mold designed so as to obtain a gel adapted to the container.

Alternatively, the gelation may be performed by forming a gel capsule containing alginic acid in accordance with a known approach (WO2010/010902). Specifically, the solution of alginate or alginic acid ester containing the cell population may be added dropwise to a solution of a cross-linking agent for gelation. The size of liquid droplets can be adjusted according to the shape of a nozzle for dropwise addition or a dropwise addition method, and by extension, the size of the gel capsule containing alginic acid can be defined. The dropwise addition method is not particularly limited and can be performed by an approach such as an air spray method, an airless spray method, or a static spray method. The size of the gel capsule containing alginic acid is not particularly limited and can be a diameter of 5 mm or smaller, 1 mm or smaller, or 500 µm or smaller.

The cross-linking agent solution can contain the cross-linking agent in an amount selected from 0.1 to 10% by weight, for example, 0.1 to 5% by weight.

In the present invention, "FGFR1 inhibitor" is a substance having inhibitory activity at least for fibroblast growth factor receptor (FGFR) 1. FGFR1 is a member of the four-pass transmembrane tyrosine kinase family (FGFR1, FGFR2, FGFR3, and FGFR4), as a receptor having high affinity for growth factors FGF1 to FGF17. FGFR1 is involved in many signaling pathways associated with the induction and pattern formation of the mesoderm, the proliferation and migration of cells, organogenesis, bone growth, etc. The FGFR1 inhibitor used in the present invention is not particularly limited as long as the FGFR1 inhibitor has FGFR1-inhibiting activity. The FGFR1 inhibitor may be a substance having the FGFR1-inhibiting activity as well as inhibitory activity for other FGFRs. In the present invention, "FGFR1 inhibitor" includes a substance having FGFR1-inhibiting activity, even if only slightly, and preferably refers to a substance that inhibits FGFR1 by 50% or more, more preferably a substance having a 50% inhibitory concentration (IC₅₀) of 1 µM or lower, further preferably 100 nM or lower, against FGFR1. A method for determining the FGFR1-inhibiting activity can be selected from known methods. Examples thereof include determination methods using EnzyChrom Kinase Assay Kit (BioAssay Systems). In the present invention, a conventionally known "FGFR1 inhibitor" may be used and can be found in patent literatures or non patent literatures. In the present invention, "FGFR1 inhibitor" can have at least FGFR1-inhibiting activity and may have inhibitory activity for other FGFRs. "FGFR1 inhibitor" of the present invention may be, for example, an FGFR2 inhibitor, an FGFR3 inhibitor, or an FGFR4 inhibitor as long as it has the FGFR1-inhibiting activity.

In the present invention, examples of the "FGFR1 inhibitor" include compounds given below (compound group D) as well as compounds having inhibitory activity for FGFR1 (or salts thereof) among compounds having structural formulas represented by the general formulas given below. The FGFR1 inhibitor is preferably a compound having a 50% inhibitory concentration (IC₅₀) of 1 µM or lower, more preferably 100 nM or lower, against FGFR1 (or a salt thereof) among compound I and compound II having structural formulas represented by the following general formulas.

### (Compound I)

Examples of compound I include compounds represented by the following formula 1: 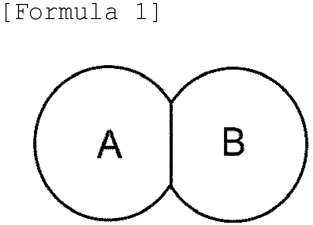 wherein ring AB shows a bicyclic heterocycle having one or more substituent(s) (ring AB may be a tricyclic heterocycle having one or more substituent(s)) or salts thereof.

Preferably, in the formula, ring AB shows a bicyclic nitrogen-containing heterocycle having three substituents.

Examples of the substituents include substituents given below and substituents of compound group D. Substituents of compound group D are preferred.

### (Compound II)

Examples of compound II include compounds represented by the following formula 2: 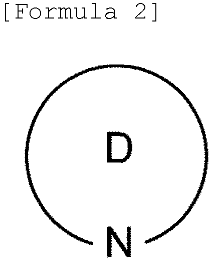
wherein ring D shows a 5- or 6-membered monocyclic nitrogen-containing heterocycle optionally having substituent(s); and
one or more nitrogen-containing heterocycle(s) other than the ring D is contained
or salts thereof.

Preferably, in the formula, ring D shows an aromatic ring containing one or two nitrogen atoms and optionally having substituent(s).

Preferably, examples of the one or more nitrogen-containing heterocycle(s) other than the ring D include piperazine optionally having substituent(s).

Examples of the substituents include substituents given below and substituents of compound group D. Substituents of compound group D are preferred.

As used herein, examples of the "heterocycle" include aromatic heterocycles and non-aromatic heterocycles each containing, as a ring-constituting atom besides carbon atom, 1 to 4 hetero atoms selected from a nitrogen atom, a sulfur atom and an oxygen atom.

As used herein, examples of the "aromatic heterocycle" include a 5- to 14-membered (preferably 5-to 10-membered) aromatic heterocycle containing, as a ring-constituting atom besides carbon atom, 1 to 4 hetero atoms selected from a nitrogen atom, a sulfur atom and an oxygen atom. Preferable examples of the "aromatic heterocycle" include 5- or 6-membered monocyclic aromatic heterocycles such as thiophene, furan, pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, triazole, tetrazole, and triazine; and 8- to 14-membered fused polycyclic (preferably bicyclic or tricyclic) aromatic heterocycles such as benzothiophene, benzofuran, benzimidazole, benzoxazole, benzisoxazole, benzothiazole, benzisothiazole, benzotriazole, imidazopyridine, thienopyridine, furopyridine, pyrrolopyridine, pyrazolopyridine, oxazolopyridine, thiazolopyridine, imidazopyrazine, imidazopyrimidine, thienopyrimidine, furopyrimidine, pyrrolopyrimidine, pyrazolopyrimidine, oxazolopyrimidine, thiazolopyrimidine, pyrazolopyrimidine, pyrazolotriazine, naphtho[2,3-b]thiophene, phenoxathiin, indole, isoindole, 1H-indazole, purine, isoquinoline, quinoline, phthalazine, naphthyridine, quinoxaline, quinazoline, cinnoline, carbazole, β-carboline, phenanthridine, acridine, phenazine, phenothiazine, and phenoxazine.

As used herein, examples of the "non-aromatic heterocycle" include a 3- to 14-membered (preferably 4-to 10-membered) non-aromatic heterocycle containing, as a ring-constituting atom besides carbon atom, 1 to 4 hetero atoms selected from a nitrogen atom, a sulfur atom and an oxygen atom. Preferable examples of the "non-aromatic heterocycle" include 3- to 8-membered monocyclic non-aromatic heterocycles such as aziridine, oxirane, thiirane, azetidine, oxetane, thietane, tetrahydrothiophene, tetrahydrofuran, pyrroline, pyrrolidine, imidazoline, imidazolidine, oxazoline, oxazolidine, pyrazoline, pyrazolidine, thiazoline, thiazolidine, tetrahydroisothiazole, tetrahydrooxazole, tetrahydroisoxazole, piperidine, piperazine, tetrahydropyridine, dihydropyridine, dihydrothiopyran, tetrahydropyrimidine, tetrahydropyridazine, dihydropyran, tetrahydropyran, tetrahydrothiopyran, morpholine, thiomorpholine, azepanine, diazepane, azepine, azocane, diazocane, oxepane; and
9- to 14-membered fused polycyclic (preferably bi or tricyclic) non-aromatic heterocycles such as dihydrobenzofuran, dihydrobenzimidazole, dihydrobenzoxazole, dihydrobenzothiazole, dihydrobenzisothiazole, dihydronaphtho[2,3-b]thiophene, tetrahydroisoquinoline, tetrahydroquinoline, 4H-quinolizine, indoline, isoindoline, tetrahydrothieno[2,3-c]pyridine, tetrahydrobenzazepine, tetrahydroquinoxaline, tetrahydrophenanthridine, hexahydrophenothiazine, hexahydrophenoxazine, tetrahydrophthalazine, tetrahydronaphthyridine, tetrahydroquinazoline, tetrahydrocinnoline, tetrahydrocarbazole, tetrahydro-β-carboline, tetrahydroacridine, tetrahydrophenazine, tetrahydrothioxanthene, octahydroisoquinoline.

As used herein, examples of the "nitrogen-containing heterocycle" include "heterocycles" containing at least one or more nitrogen atom(s) as a ring-constituting atom.

Hereinafter, each substituent used herein will be defined in detail. Each substituent is as defined below unless otherwise specified.

As used herein, examples of the "halogen atom" include fluorine, chloride, bromine, and iodine.

As used herein, examples of the "C₁₋₆ alkyl group" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl.

As used herein, examples of the "optionally halogenated C₁₋₆ alkyl group" include a C₁₋₆ alkyl group optionally having 1 to 7, preferably 1 to 5 halogen atoms. Specific examples thereof include methyl, chloromethyl, difluoromethyl, trichloromethyl, trifluoromethyl, ethyl, 2-bromoethyl, 2,2,2-trifluoroethyl, tetrafluoroethyl, pentafluoroethyl, propyl, 2,2-difluoropropyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, and 6,6,6-trifluorohexyl.

As used herein, examples of the "C₂₋₆ alkenyl group" include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, and 5-hexenyl.

As used herein, examples of the "C₂₋₆ alkynyl group" include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, and 4-methyl-2-pentynyl.

As used herein, examples of the "C₃₋₁₀ cycloalkyl group" include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, and adamantyl.

As used herein, examples of the "optionally halogenated C₃₋₁₀ cycloalkyl group" include a C₃₋₁₀ cycloalkyl group optionally having 1 to 7, preferably 1 to 5 halogen atoms. Specific examples thereof include cyclopropyl, 2,2-difluorocyclopropyl, 2,3-difluorocyclopropyl, cyclobutyl, difluorocyclobutyl, cyclopentyl, cyclohexyl, cycloheptyl, and cyclooctyl.

As used herein, examples of the "C₃₋₁₀ cycloalkenyl group" include cyclopropenyl, cyclobutenyl, cyclopentenyl, cyclohexenyl, cycloheptenyl, and cyclooctenyl.

As used herein, examples of the "C₆₋₁₄ aryl group" include phenyl, 1-naphthyl, 2-naphthyl, 1-anthryl, 2-anthryl, and 9-anthryl.

As used herein, examples of the "C₇₋₁₆ aralkyl group" include benzyl, phenethyl, naphthylmethyl, and phenylpropyl.

As used herein, examples of the "C₁₋₆ alkoxy group" include methoxy, ethoxy, propoxy, isopropoxy, butoxy, isobutoxy, sec-butoxy, tert-butoxy, pentyloxy, and hexyloxy.

As used herein, examples of the "optionally halogenated C₁₋₆ alkoxy group" include a C₁₋₆ alkoxy group optionally having 1 to 7, preferably 1 to 5 halogen atoms. Specific examples thereof include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, and hexyloxy.

As used herein, examples of the "C₃₋₁₀ cycloalkyloxy group" include cyclopropyloxy, cyclobutyloxy, cyclopentyloxy, cyclohexyloxy, cycloheptyloxy, and cyclooctyloxy.

As used herein, examples of the "C₁₋₆ alkylthio group" include methylthio, ethylthio, propylthio, isopropylthio, butylthio, sec-butylthio, tert-butylthio, pentylthio, and hexylthio.

As used herein, examples of the "optionally halogenated C₁₋₆ alkylthio group" include a C₁₋₆ alkylthio group optionally having 1 to 7, preferably 1 to 5 halogen atoms. Specific examples thereof include methylthio, difluoromethylthio, trifluoromethylthio, ethylthio, propylthio, isopropylthio, butylthio, 4,4,4-trifluorobutylthio, pentylthio, and hexylthio.

As used herein, examples of the "C₁₋₆ alkyl-carbonyl group" include acetyl, propanoyl, butanoyl, 2-methylpropanoyl, pentanoyl, 3-methylbutanoyl, 2-methylbutanoyl, 2,2-dimethylpropanoyl, hexanoyl, and heptanoyl.

As used herein, examples of the "optionally halogenated C₁₋₆ alkyl-carbonyl group" include a C₁₋₆ alkyl-carbonyl group optionally having 1 to 7, preferably 1 to 5 halogen atoms. Specific examples thereof include acetyl, chloroacetyl, trifluoroacetyl, trichloroacetyl, propanoyl, butanoyl, pentanoyl, and hexanoyl.

As used herein, examples of the "C₁₋₆ alkoxy-carbonyl group" include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, and hexyloxycarbonyl.

As used herein, examples of the "C₆₋₁₄ aryl-carbonyl group" include benzoyl, 1-naphthoyl, and 2-naphthoyl.

As used herein, examples of the "C₇₋₁₆ aralkyl-carbonyl group" include phenylacetyl and phenylpropionyl.

As used herein, examples of the "5- to 14-membered aromatic heterocyclylcarbonyl group" include nicotinoyl, isonicotinoyl, thenoyl, and furoyl.

As used herein, examples of the "3- to 14-membered non-aromatic heterocyclylcarbonyl group" include morpholinylcarbonyl, piperidinylcarbonyl, and pyrrolidinylcarbonyl.

As used herein, examples of the "mono- or di-C₁₋₆ alkyl-carbamoyl group" include methylcarbamoyl, ethylcarbamoyl, dimethylcarbamoyl, diethylcarbamoyl, and N-ethyl-N-methylcarbamoyl.

As used herein, examples of the "mono- or di-C₇₋₁₆ aralkyl-carbamoyl group" include benzylcarbamoyl and phenethylcarbamoyl.

As used herein, examples of the "C₁₋₆ alkylsulfonyl group" include methylsulfonyl, ethylsulfonyl, propylsulfonyl, isopropylsulfonyl, butylsulfonyl, sec-butylsulfonyl, and tert-butylsulfonyl.

As used herein, examples of the "optionally halogenated C₁₋₆ alkylsulfonyl group" include a C₁₋₆ alkylsulfonyl group optionally having 1 to 7, preferably 1 to 5 halogen atoms. Specific examples thereof include methylsulfonyl, difluoromethylsulfonyl, trifluoromethylsulfonyl, ethylsulfonyl, propylsulfonyl, isopropylsulfonyl, butylsulfonyl, 4,4,4-trifluorobutylsulfonyl, pentylsulfonyl, and hexylsulfonyl.

As used herein, examples of the "C₆₋₁₄ arylsulfonyl group" include phenylsulfonyl, 1-naphthylsulfonyl, and 2-naphthylsulfonyl.

As used herein, examples of the "substituent" include a halogen atom, a cyano group, a nitro group, an optionally substituted hydrocarbon group, an optionally substituted heterocyclic group, an acyl group, an optionally substituted amino group, an optionally substituted carbamoyl group, an optionally substituted thiocarbamoyl group, an optionally substituted sulfamoyl group, an optionally substituted hydroxy group, an optionally substituted sulfanyl (SH) group and an optionally substituted silyl group.

In the present specification, examples of the "hydrocarbon group" (including "hydrocarbon group" of "optionally substituted hydrocarbon group") include a C₁₋₆ alkyl group, a C₂₋₆ alkenyl group, a C₂₋₆ alkynyl group, a C₃₋₁₀ cycloalkyl group, a C₃₋₁₀ cycloalkenyl group, a C₆₋₁₄ aryl group and a C₇₋₁₆ aralkyl group.

In the present specification, examples of the "optionally substituted hydrocarbon group" include a hydrocarbon group optionally having substituent(s) selected from the following substituent group A.

### [substituent group A]

(1) a halogen atom,
(2) a nitro group,
(3) a cyano group,
(4) an oxo group,
(5) a hydroxy group,
(6) an optionally halogenated C₁₋₆ alkoxy group,
(7) a C₆₋₁₄ aryloxy group (e.g., phenoxy, naphthoxy),
(8) a C₇₋₁₆ aralkyloxy group (e.g., benzyloxy),
(9) a 5- to 14-membered aromatic heterocyclyloxy group (e.g., pyridyloxy),
(10) a 3- to 14-membered non-aromatic heterocyclyloxy group (e.g., morpholinyloxy, piperidinyloxy),
(11) a C₁₋₆ alkyl-carbonyloxy group (e.g., acetoxy, propanoyloxy),
(12) a C₆₋₁₄ aryl-carbonyloxy group (e.g., benzoyloxy, 1-naphthoyloxy, 2-naphthoyloxy),
(13) a C₁₋₆ alkoxy-carbonyloxy group (e.g., methoxycarbonyloxy, ethoxycarbonyloxy, propoxycarbonyloxy, butoxycarbonyloxy),
(14) a mono- or di-C₁₋₆ alkyl-carbamoyloxy group (e.g., methylcarbamoyloxy, ethylcarbamoyloxy, dimethylcarbamoyloxy, diethylcarbamoyloxy),
(15) a C₆₋₁₄ aryl-carbamoyloxy group (e.g., phenylcarbamoyloxy, naphthylcarbamoyloxy),
(16) a 5- to 14-membered aromatic heterocyclylcarbonyloxy group (e.g., nicotinoyloxy),
(17) a 3- to 14-membered non-aromatic heterocyclylcarbonyloxy group (e.g., morpholinylcarbonyloxy, piperidinylcarbonyloxy),
(18) an optionally halogenated C₁₋₆ alkylsulfonyloxy group (e.g., methylsulfonyloxy, trifluoromethylsulfonyloxy),
(19) a C₆₋₁₄ arylsulfonyloxy group optionally substituted by a C₁₋₆ alkyl group (e.g., phenylsulfonyloxy, toluenesulfonyloxy),
(20) an optionally halogenated C₁₋₆ alkylthio group,
(21) a 5- to 14-membered aromatic heterocyclic group,
(22) a 3- to 14-membered non-aromatic heterocyclic group,
(23) a formyl group,
(24) a carboxy group,
(25) an optionally halogenated C₁₋₆ alkyl-carbonyl group,
(26) a C₆₋₁₄ aryl-carbonyl group,
(27) a 5- to 14-membered aromatic heterocyclylcarbonyl group,
(28) a 3- to 14-membered non-aromatic heterocyclylcarbonyl group,
(29) a C₁₋₆ alkoxy-carbonyl group,
(30) a C₆₋₁₄ aryloxy-carbonyl group (e.g., phenyloxycarbonyl, 1-naphthyloxycarbonyl, 2-naphthyloxycarbonyl),
(31) a C₇₋₁₆ aralkyloxy-carbonyl group (e.g., benzyloxycarbonyl, phenethyloxycarbonyl),
(32) a carbamoyl group,
(33) a thiocarbamoyl group,
(34) a mono- or di-C₁₋₆ alkyl-carbamoyl group,
(35) a C₆₋₁₄ aryl-carbamoyl group (e.g., phenylcarbamoyl),
(36) a 5- to 14-membered aromatic heterocyclylcarbamoyl group (e.g., pyridylcarbamoyl, thienylcarbamoyl),
(37) a 3- to 14-membered non-aromatic heterocyclylcarbamoyl group (e.g., morpholinylcarbamoyl, piperidinylcarbamoyl),
(38) an optionally halogenated C₁₋₆ alkylsulfonyl group,
(39) a C₆₋₁₄ arylsulfonyl group,
(40) a 5- to 14-membered aromatic heterocyclylsulfonyl group (e.g., pyridylsulfonyl, thienylsulfonyl),
(41) an optionally halogenated C₁₋₆ alkylsulfinyl group,
(42) a C₆₋₁₄ arylsulfinyl group (e.g., phenylsulfinyl, 1-naphthylsulfinyl, 2-naphthylsulfinyl),
(43) a 5- to 14-membered aromatic heterocyclylsulfinyl group (e.g., pyridylsulfinyl, thienylsulfinyl),
(44) an amino group,
(45) a mono- or di-C₁₋₆ alkylamino group (e.g., methylamino, ethylamino, propylamino, isopropylamino, butylamino, dimethylamino, diethylamino, dipropylamino, dibutylamino, N-ethyl-N-methylamino),
(46) a mono- or di-C₆₋₁₄ arylamino group (e.g., phenylamino),
(47) a 5- to 14-membered aromatic heterocyclylamino group (e.g., pyridylamino),
(48) a C₇₋₁₆ aralkylamino group (e.g., benzylamino),
(49) a formylamino group,
(50) a C₁₋₆ alkyl-carbonylamino group (e.g., acetylamino, propanoylamino, butanoylamino),
(51) a (C₁₋₆ alkyl) (C₁₋₆ alkyl-carbonyl) amino group (e.g., N-acetyl-N-methylamino),
(52) a C₆₋₁₄ aryl-carbonylamino group (e.g., phenylcarbonylamino, naphthylcarbonylamino),
(53) a C₁₋₆ alkoxy-carbonylamino group (e.g., methoxycarbonylamino, ethoxycarbonylamino, propoxycarbonylamino, butoxycarbonylamino, tert-butoxycarbonylamino),
(54) a C₇₋₁₆ aralkyloxy-carbonylamino group (e.g., benzyloxycarbonylamino),
(55) a C₁₋₆ alkylsulfonylamino group (e.g., methylsulfonylamino, ethylsulfonylamino),
(56) a C₆₋₁₄ arylsulfonylamino group optionally substituted by a C₁₋₆ alkyl group (e.g., phenylsulfonylamino, toluenesulfonylamino),
(57) an optionally halogenated C₁₋₆ alkyl group,
(58) a C₂₋₆ alkenyl group,
(59) a C₂₋₆ alkynyl group,
(60) a C₃₋₁₀ cycloalkyl group,
(61) a C₃₋₁₀ cycloalkenyl group and
(62) a C₆₋₁₄ aryl group.

The number of the above-mentioned substituents in the "optionally substituted hydrocarbon group" is, for example, 1 to 5, preferably 1 to 3. When the number of the substituents is two or more, the respective substituents may be the same or different.

In the present specification, examples of the "heterocyclic group" (including "heterocyclic group" of "optionally substituted heterocyclic group") include (i) an aromatic heterocyclic group, (ii) a non-aromatic heterocyclic group and (iii) a 7- to 10-membered bridged heterocyclic group, each containing, as a ring-constituting atom besides carbon atom, 1 to 4 hetero atoms selected from a nitrogen atom, a sulfur atom and an oxygen atom.

In the present specification, examples of the "aromatic heterocyclic group" (including "5- to 14-membered aromatic heterocyclic group") include a 5- to 14-membered (preferably 5- to 10-membered) aromatic heterocyclic group containing, as a ring-constituting atom besides carbon atom, 1 to 4 hetero atoms selected from a nitrogen atom, a sulfur atom and an oxygen atom.

Preferable examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl and the like; and 8- to 14-membered fused polycyclic (preferably bi or tricyclic) aromatic heterocyclic groups such as benzothiophenyl, benzofuranyl, benzimidazolyl, benzoxazolyl, benzisoxazolyl, benzothiazolyl, benzisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acridinyl, phenazinyl, phenothiazinyl, phenoxazinyl and the like.

In the present specification, examples of the "non-aromatic heterocyclic group" (including "3- to 14-membered non-aromatic heterocyclic group") include a 3-to 14-membered (preferably 4- to 10-membered) non-aromatic heterocyclic group containing, as a ring-constituting atom besides carbon atom, 1 to 4 hetero atoms selected from a nitrogen atom, a sulfur atom and an oxygen atom.

Preferable examples of the "non-aromatic heterocyclic group" include 3- to 8-membered monocyclic non-aromatic heterocyclic groups such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisooxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl and the like; and 9- to 14-membered fused polycyclic (preferably bi or tricyclic) non-aromatic heterocyclic groups such as dihydrobenzofuranyl, dihydrobenzimidazolyl, dihydrobenzoxazolyl, dihydrobenzothiazolyl, dihydrobenzisothiazolyl, dihydronaphtho[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacrydinyl, tetrahydrophenazinyl, tetrahydrothioxanthenyl, octahydroisoquinolyl and the like.

In the present specification, preferable examples of the "7- to 10-membered bridged heterocyclic group" include quinuclidinyl and 7-azabicyclo[2.2.1]heptanyl.

In the present specification, examples of the "nitrogen-containing heterocyclic group" include a "heterocyclic group" containing at least one nitrogen atom as a ring-constituting atom.

In the present specification, examples of the "optionally substituted heterocyclic group" include a heterocyclic group optionally having substituent(s) selected from the aforementioned substituent group A.

The number of the substituents in the "optionally substituted heterocyclic group" is, for example, 1 to 3. When the number of the substituents is two or more, the respective substituents may be the same or different.

In the present specification, examples of the "acyl group" include a formyl group, a carboxy group, a carbamoyl group, a thiocarbamoyl group, a sulfino group, a sulfo group, a sulfamoyl group and a phosphono group, each optionally having "1 or 2 substituents selected from a C₁₋₆ alkyl group, a C₂₋₆ alkenyl group, a C₃₋₁₀ cycloalkyl group, a C₃₋₁₀ cycloalkenyl group, a C₆₋₁₄ aryl group, a C₇₋₁₆ aralkyl group, a 5- to 14-membered aromatic heterocyclic group and a 3- to 14-membered non-aromatic heterocyclic group, each of which optionally has 1 to 3 substituents selected from a halogen atom, an optionally halogenated C₁₋₆ alkoxy group, a hydroxy group, a nitro group, a cyano group, an amino group and a carbamoyl group".

Examples of the "acyl group" also include a hydrocarbon-sulfonyl group, a heterocyclylsulfonyl group, a hydrocarbon-sulfinyl group and a heterocyclylsulfinyl group.

Here, the hydrocarbon-sulfonyl group means a hydrocarbon group-bonded sulfonyl group, the heterocyclylsulfonyl group means a heterocyclic group-bonded sulfonyl group, the hydrocarbon-sulfinyl group means a hydrocarbon group-bonded sulfinyl group and the heterocyclylsulfinyl group means a heterocyclic group-bonded sulfinyl group.

Preferable examples of the "acyl group" include a formyl group, a carboxy group, a C₁₋₆ alkyl-carbonyl group, a C₂₋₆ alkenyl-carbonyl group (e.g., crotonoyl), a C₃₋₁₀ cycloalkyl-carbonyl group (e.g., cyclobutanecarbonyl, cyclopentanecarbonyl, cyclohexanecarbonyl, cycloheptanecarbonyl), a C₃₋₁₀ cycloalkenyl-carbonyl group (e.g., 2-cyclohexenecarbonyl), a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a C₆₋₁₄ aryloxy-carbonyl group (e.g., phenyloxycarbonyl, naphthyloxycarbonyl), a C₇₋₁₆ aralkyloxy-carbonyl group (e.g., benzyloxycarbonyl, phenethyloxycarbonyl), a carbamoyl group, a mono- or di-C₁₋₆ alkyl-carbamoyl group, a mono- or di-C₂₋₆ alkenyl-carbamoyl group (e.g., diallylcarbamoyl), a mono- or di-C₃₋₁₀ cycloalkyl-carbamoyl group (e.g., cyclopropylcarbamoyl), a mono- or di-C₆₋₁₄ aryl-carbamoyl group (e.g., phenylcarbamoyl), a mono- or di-C₇₋₁₆ aralkyl-carbamoyl group, a 5- to 14-membered aromatic heterocyclylcarbamoyl group (e.g., pyridylcarbamoyl), a thiocarbamoyl group, a mono- or di-C₁₋₆ alkyl-thiocarbamoyl group (e.g., methylthiocarbamoyl, N-ethyl-N-methylthiocarbamoyl), a mono- or di-C₂₋₆ alkenyl-thiocarbamoyl group (e.g., diallylthiocarbamoyl), a mono- or di-C₃₋₁₀ cycloalkyl-thiocarbamoyl group (e.g., cyclopropylthiocarbamoyl, cyclohexylthiocarbamoyl), a mono- or di-C₆₋₁₄ aryl-thiocarbamoyl group (e.g., phenylthiocarbamoyl), a mono- or di-C₇₋₁₆ aralkyl-thiocarbamoyl group (e.g., benzylthiocarbamoyl, phenethylthiocarbamoyl), a 5- to 14-membered aromatic heterocyclylthiocarbamoyl group (e.g., pyridylthiocarbamoyl), a sulfino group, a C₁₋₆ alkylsulfinyl group (e.g., methylsulfinyl, ethylsulfinyl), a sulfo group, a C₁₋₆ alkylsulfonyl group, a C₆₋₁₄ arylsulfonyl group, a phosphono group and a mono- or di-C₁₋₆ alkylphosphono group (e.g., dimethylphosphono, diethylphosphono, diisopropylphosphono, dibutylphosphono).

In the present specification, examples of the "optionally substituted amino group" include an amino group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group, a mono- or di-C₇₋₁₆ aralkyl-carbamoyl group, a C₁₋₆ alkylsulfonyl group and a C₆₋₁₄ arylsulfonyl group, each of which optionally has 1 to 3 substituents selected from substituent group A".

Preferable examples of the optionally substituted amino group include an amino group, a mono- or di-(optionally halogenated C₁₋₆ alkyl)amino group (e.g., methylamino, trifluoromethylamino, dimethylamino, ethylamino, diethylamino, propylamino, dibutylamino), a mono- or di-C₂₋₆ alkenylamino group (e.g., diallylamino), a mono- or di -C₃₋₁₀ cycloalkylamino group (e.g., cyclopropylamino, cyclohexylamino), a mono- or di-C₆₋₁₄ arylamino group (e.g., phenylamino), a mono- or di-C₇₋₁₆ aralkylamino group (e.g., benzylamino, dibenzylamino), a mono- or di-(optionally halogenated C₁₋₆ alkyl)-carbonylamino group (e.g., acetylamino, propionylamino), a mono- or di-C₆₋₁₄ aryl-carbonylamino group (e.g., benzoylamino), a mono- or di-C₇₋₁₆ aralkyl-carbonylamino group (e.g., benzylcarbonylamino), a mono- or di-5- to 14-membered aromatic heterocyclylcarbonylamino group (e.g., nicotinoylamino, isonicotinoylamino), a mono- or di-3- to 14-membered non-aromatic heterocyclylcarbonylamino group (e.g., piperidinylcarbonylamino), a mono- or di-C₁₋₆ alkoxy-carbonylamino group (e.g., tert-butoxycarbonylamino), a 5- to 14-membered aromatic heterocyclylamino group (e.g., pyridylamino), a carbamoylamino group, a (mono- or di-C₁₋₆ alkyl-carbamoyl)amino group (e.g., methylcarbamoylamino), a (mono- or di-C₇₋₁₆ aralkyl-carbamoyl) amino group (e.g., benzylcarbamoylamino), a C₁₋₆ alkylsulfonylamino group (e.g., methylsulfonylamino, ethylsulfonylamino), a C₆₋₁₄ arylsulfonylamino group (e.g., phenylsulfonylamino), a (C₁₋₆ alkyl) (C₁₋₆ alkyl-carbonyl) amino group (e.g., N-acetyl-N-methylamino) and a (C₁₋₆ alkyl) (C₆₋₁₄ aryl-carbonyl)amino group (e.g., N-benzoyl-N-methylamino).

In the present specification, examples of the "optionally substituted carbamoyl group" include a carbamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted carbamoyl group include a carbamoyl group, a mono- or di-C₁₋₆ alkyl-carbamoyl group, a mono- or di-C₂₋₆ alkenyl-carbamoyl group (e.g., diallylcarbamoyl), a mono- or di-C₃₋₁₀ cycloalkyl-carbamoyl group (e.g., cyclopropylcarbamoyl, cyclohexylcarbamoyl), a mono- or di-C₆₋₁₄ aryl-carbamoyl group (e.g., phenylcarbamoyl), a mono- or di-C₇₋₁₆ aralkyl-carbamoyl group, a mono- or di-C₁₋₆ alkyl-carbonyl-carbamoyl group (e.g., acetylcarbamoyl, propionylcarbamoyl), a mono- or di-C₆₋₁₄ aryl-carbonyl-carbamoyl group (e.g., benzoylcarbamoyl) and a 5- to 14-membered aromatic heterocyclylcarbamoyl group (e.g., pyridylcarbamoyl).

In the present specification, examples of the "optionally substituted thiocarbamoyl group" include a thiocarbamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted thiocarbamoyl group include a thiocarbamoyl group, a mono- or di-C₁₋₆ alkyl-thiocarbamoyl group (e.g., methylthiocarbamoyl, ethylthiocarbamoyl, dimethylthiocarbamoyl, diethylthiocarbamoyl, N-ethyl-N-methylthiocarbamoyl), a mono- or di-C₂₋₆ alkenyl-thiocarbamoyl group (e.g., diallylthiocarbamoyl), a mono- or di-C₃₋₁₀ cycloalkyl-thiocarbamoyl group (e.g., cyclopropylthiocarbamoyl, cyclohexylthiocarbamoyl), a mono- or di-C₆₋₁₄ aryl-thiocarbamoyl group (e.g., phenylthiocarbamoyl), a mono- or di-C₇₋₁₆ aralkyl-thiocarbamoyl group (e.g., benzylthiocarbamoyl, phenethylthiocarbamoyl), a mono- or di-C₁₋₆ alkyl-carbonyl-thiocarbamoyl group (e.g., acetylthiocarbamoyl, propionylthiocarbamoyl), a mono- or di-C₆₋₁₄ aryl-carbonyl-thiocarbamoyl group (e.g., benzoylthiocarbamoyl) and a 5- to 14-membered aromatic heterocyclylthiocarbamoyl group (e.g., pyridylthiocarbamoyl).

In the present specification, examples of the "optionally substituted sulfamoyl group" include a sulfamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted sulfamoyl group include a sulfamoyl group, a mono- or di-C₁₋₆ alkyl-sulfamoyl group (e.g., methylsulfamoyl, ethylsulfamoyl, dimethylsulfamoyl, diethylsulfamoyl, N-ethyl-N-methylsulfamoyl), a mono- or di-C₂₋₆ alkenyl-sulfamoyl group (e.g., diallylsulfamoyl), a mono- or di-C₃₋₁₀ cycloalkyl-sulfamoyl group (e.g., cyclopropylsulfamoyl, cyclohexylsulfamoyl), a mono- or di-C₆₋₁₄ aryl-sulfamoyl group (e.g., phenylsulfamoyl), a mono- or di-C₇₋₁₆ aralkyl-sulfamoyl group (e.g., benzylsulfamoyl, phenethylsulfamoyl), a mono- or di-C₁₋₆ alkyl-carbonyl-sulfamoyl group (e.g., acetylsulfamoyl, propionylsulfamoyl), a mono- or di-C₆₋₁₄ aryl-carbonyl-sulfamoyl group (e.g., benzoylsulfamoyl) and a 5- to 14-membered aromatic heterocyclylsulfamoyl group (e.g., pyridylsulfamoyl).

In the present specification, examples of the "optionally substituted hydroxy group" include a hydroxyl group optionally having "a substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group, a mono- or di-C₇₋₁₆ aralkyl-carbamoyl group, a C₁₋₆ alkylsulfonyl group and a C₆₋₁₄ arylsulfonyl group, each of which optionally has 1 to 3 substituents selected from substituent group A".

Preferable examples of the optionally substituted hydroxy group include a hydroxy group, a C₁₋₆ alkoxy group, a C₂₋₆ alkenyloxy group (e.g., allyloxy, 2-butenyloxy, 2-pentenyloxy, 3-hexenyloxy), a C₃₋₁₀ cycloalkyloxy group (e.g., cyclohexyloxy), a C₆₋₁₄ aryloxy group (e.g., phenoxy, naphthyloxy), a C₇₋₁₆ aralkyloxy group (e.g., benzyloxy, phenethyloxy), a C₁₋₆ alkyl-carbonyloxy group (e.g., acetyloxy, propionyloxy, butyryloxy, isobutyryloxy, pivaloyloxy), a C₆₋₁₄ aryl-carbonyloxy group (e.g., benzoyloxy), a C₇₋₁₆ aralkyl-carbonyloxy group (e.g., benzylcarbonyloxy), a 5- to 14-membered aromatic heterocyclylcarbonyloxy group (e.g., nicotinoyloxy), a 3- to 14-membered non-aromatic heterocyclylcarbonyloxy group (e.g., piperidinylcarbonyloxy), a C₁₋₆ alkoxy-carbonyloxy group (e.g., tert-butoxycarbonyloxy), a 5- to 14-membered aromatic heterocyclyloxy group (e.g., pyridyloxy), a carbamoyloxy group, a C₁₋₆ alkyl-carbamoyloxy group (e.g., methylcarbamoyloxy), a C₇₋₁₆ aralkyl-carbamoyloxy group (e.g., benzylcarbamoyloxy), a C₁₋₆ alkylsulfonyloxy group (e.g., methylsulfonyloxy, ethylsulfonyloxy) and a C₆₋₁₄ arylsulfonyloxy group (e.g., phenylsulfonyloxy).

In the present specification, examples of the "optionally substituted sulfanyl group" include a sulfanyl group optionally having "a substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group and a 5- to 14-membered aromatic heterocyclic group, each of which optionally has 1 to 3 substituents selected from substituent group A" and a halogenated sulfanyl group.

Preferable examples of the optionally substituted sulfanyl group include a sulfanyl (-SH) group, a C₁₋₆ alkylthio group, a C₂₋₆ alkenylthio group (e.g., allylthio, 2-butenylthio, 2-pentenylthio, 3-hexenylthio), a C₃₋₁₀ cycloalkylthio group (e.g., cyclohexylthio), a C₆₋₁₄ arylthio group (e.g., phenylthio, naphthylthio), a C₇₋₁₆ aralkylthio group (e.g., benzylthio, phenethylthio), a C₁₋₆ alkyl-carbonylthio group (e.g., acetylthio, propionylthio, butyrylthio, isobutyrylthio, pivaloylthio), a C₆₋₁₄ aryl-carbonylthio group (e.g., benzoylthio), a 5- to 14-membered aromatic heterocyclylthio group (e.g., pyridylthio) and a halogenated thio group (e.g., pentafluorothio).

In the present specification, examples of the "optionally substituted silyl group" include a silyl group optionally having "1 to 3 substituents selected from a C₁₋₆ alkyl group, a C₂₋₆ alkenyl group, a C₃₋₁₀ cycloalkyl group, a C₆₋₁₄ aryl group and a C₇₋₁₆ aralkyl group, each of which optionally has 1 to 3 substituents selected from substituent group A".

Preferable examples of the optionally substituted silyl group include a tri-C₁₋₆ alkylsilyl group (e.g., trimethylsilyl, tert-butyl(dimethyl)silyl).

More specifically, examples of the FGFR1 inhibitor that may be used in the present invention include PD-166866 (1-[2-amino-6-(3,5-dimethoxyphenyl)-pyrido(2,3-d)pyrimidin-7-yl]-3-tert-butylurea: CAS No.: 192705-79-6) (in the present specification, this compound is also referred to as "CAS192705-79-6"), E-3810 (CAS No.: 1058137-23-7), PD-173074 (CAS No.: 219580-11-7), FGFR4-IN-1 (CAS No.: 1708971-72-5), FGFR-IN-1 (CAS No.: 1448169-71-8), FIIN-2 (CAS No.: 1633044-56-0), AZD4547 (CAS No.: 1035270-39-3), FIIN-3 (CAS No.: 1637735-84-2), NVP-BGJ398 (CAS No.: 1310746-10-1), NVP-BGJ398 (CAS No.: 872511-34-7), CH5183284 (CAS No.: 1265229-25-1), Derazantinib (CAS No.: 1234356-69-4), Derazantinib Racemate, Ferulic acid (CAS No.: 1135-24-6), SSR128129E (CAS No.: 848318-25-2), SSR128129E free acid (CAS No.: 848463-13-8), Erdafitinib (CAS No.: 1346242-81-6), BLU9931 (CAS No.: 1538604-68-0), PRN1371 (CAS No.: 1802929-43-6), S49076 (CAS No.: 1265965-22-7), LY2874455 (CAS No.: 1254473-64-7), Linsitinib (CAS No.: 867160-71-2), Dovitinib (CAS No.: 405169-16-6), Anlotinib (CAS No.: 1058156-90-3), Brivanib (CAS No.: 649735-46-6), Derazantinib (CAS No.: 1234356-69-4), Anlotinib Dihydrochloride (CAS No.: 1360460-82-7), ACTB-1003 (CAS No.: 939805-30-8), BLU-554 (CAS No.: 1707289-21-1), Rogaratinib (CAS No.: 1443530-05-9), BIBF 1120 esylate (CAS No.: 656247-18-6), TG 100572 Hydrochloride (CAS No.: 867331-64-4), ENMD-2076 (CAS No.: 934353-76-1), Brivanib alaninate (CAS No.: 649735-63-7), TG 100572 (CAS No.: 867334-05-2), BIBF 1120 (CAS No.: 656247-17-5), ENMD-2076 Tartrate (CAS No.: 1291074-87-7), TSU-68 (CAS No.: 252916-29-3), Ponatinib (CAS No.: 943319-70-8), Sulfatinib (CAS No.: 1308672-74-3), LY2784544 (CAS No.: 1229236-86-5), Dovitinib lactate (CAS No.: 692737-80-7), SU 5402 (CAS No.: 215543-92-3), FGF-401 (CAS No.: 1708971-55-4), Tyrosine kinase-IN-1 (CAS No.: 705946-27-6), PP58 (CAS No.: 212391-58-7), TG 100801 Hydrochloride (CAS No.: 1018069-81-2), Crenolanib (CAS No.: 670220-88-9), TG 100801 (CAS No.: 867331-82-6), Pazopanib Hydrochloride (CAS No.: 635702-64-6), Pazopanib (CAS No.: 444731-52-6), PD168393 (CAS No.: 194423-15-9), Apatinib (CAS No.: 1218779-75-9), Palbociclib isethionate (CAS No.: 827022-33-3), Foretinib (CAS No.: 849217-64-7), Lenvatinib (CAS No.: 417716-92-8), Tandutinib (CAS No.: 387867-13-2), and salts thereof (these compounds are referred to as compound group D). These compounds may each have one or more substituent(s) selected from those described above as long as the compound have FGFR1-inhibiting activity, preferably a 50% inhibitory concentration (IC₅₀) of 100 nM or lower against FGFR1.

The substructure (substituent, ring, etc.) of each of these compounds may be partially converted as long as the compound have FGFR1-inhibiting activity, preferably a 50% inhibitory concentration (IC₅₀) of 100 nM or lower against FGFR1.

In the present invention, the FGFR1 inhibitor is preferably CAS192705-79-6 (1-[2-amino-6-(3,5-dimethoxyphenyl)-pyrido(2,3-d)pyrimidin-7-yl]-3-tert-butylurea: CAS No.: 192705-79-6), E-3810 (CAS No.: 1058137-23-7), or PD173074 (CAS No.: 219580-11-7).

CAS192705-79-6 has been found to have inhibitory activity for FGFR1, FGFR2, FGFR3 and FGFR4 by the following test: fluorescent probes binding to FGFR1, FGFR2, FGFR3 and FGFR4 prepared as recombinant proteins were allowed to act in the presence or absence of CAS192705-79-6, and fluorescent signals were detected using a plate reader. The inhibitory activity of CAS192705-79-6 for each protein (pIC₅₀) was calculated from change in fluorescent signal between the presence and absence of CAS192705-79-6.

The FGFR1 inhibitor is not limited to the compounds described above, and an antisense oligonucleotide or siRNA against FGFR1 mRNA, an antibody binding to FGFR1, a dominant negative FGFR1 mutant, or the like can also be used as the FGFR1 inhibitor. Such an FGFR1 inhibitor is commercially available or can be synthesized according to a known method.

Each compound mentioned above or a salt thereof can be used as the FGFR1 inhibitor. The amount of the EGFR1 inhibitor added to the medium is appropriately determined according to the compound or the salt thereof used and is usually about 0.00001 µM to 100 µM, preferably 0.01 µM to 10 µM.

The endocrine progenitor cell population or the cell population at a later stage of differentiation can be treated with the FGFR1 inhibitor by the coexistence of the cell population with the FGFR1 inhibitor. For example, the treatment can be performed by culturing the cell population in a medium supplemented with the FGFR1 inhibitor. The FGFR1 inhibitor can be contained in any amount capable of inhibiting FGFR1 activity in the medium, and can be contained in an amount of, for example, 10 µM or less or 5 µM or less, preferably in an amount of less than 5 µM, less than 4 µM, less than 3 µM, or less than 2 µM. The lower limit of the amount of the FGFR1 inhibitor added is not particularly limited and can be 0.1 µM or more, preferably 0.5 µM or more. The amount of the FGFR1 inhibitor added is preferably less than 5 µM and 0.1 µM or more, more preferably less than 5 µM and 0.5 µM or more. The culture in the presence of the FGFR1 inhibitor can be performed for at least 12 hours, preferably 24 hours or longer, 2 days or longer, 4 days or longer, 8 days or longer, 10 days or longer, or 15 days or longer. The culture in the presence of the FGFR1 inhibitor is preferably performed for 4 days or longer. The medium may be replaced during the period of treatment with the FGFR1 inhibitor and can be replaced with a medium supplemented with the FGFR1 inhibitor, having the same or different composition as or from that before the replacement, according to the culture schedule.

In the present invention, the proportion of Ki67-positive cells in the cells to be treated with the FGFR1 inhibitor (starting material cells) is not particularly limited and may be 1% or more, 5% or more, 10% or more, 20% or more, 30% or more, 40% or more, 50% or more, 60% or more, 70% or more, 80% or more, or 90% or more.

In the present invention, the proportion of alkaline phosphatase-positive cells in the cells to be treated with the FGFR1 inhibitor (starting material cells) is not particularly limited and may be 10% or less, 5% or less, 1% or less, or 0.5% or less.

The endocrine progenitor cell population or the cell population at a later stage of differentiation can be subjected to the step of further differentiation into the cell population of interest (for example, an insulin-producing cell population or a pancreatic β cell population), in addition to being treated with the FGFR1 inhibitor. As used herein, "in addition to being treated with the FGFR1 inhibitor" includes the case of performing the step of treatment with the FGFR1 inhibitor and the step of differentiation at the same time, the case of treating the cell population with the FGFR1 inhibitor, followed by the step of differentiation, and the case of subjecting the cell population to the step of differentiation, followed by the step of treatment with the FGFR1 inhibitor. Thus, the medium for use in the treatment with the FGFR1 inhibitor and the medium for use in the differentiation of the cell population may be separate media, or the medium for use in the step of differentiation may be further supplemented with the FGFR1 inhibitor.

In one embodiment, the FGFR1 inhibitor can be contained in a medium for use in the step of differentiating endocrine progenitor cells into insulin-producing cells and can be more preferably contained in a medium for use in the step of further differentiating (for example, for about last 4 to 15 days, preferably about last 4 to 7 days, of the step 6) a population of an early insulin-producing cells obtained by the induction of differentiation of an endocrine progenitor cell population or cells at a later stage of differentiation. In the case of treating an endocrine progenitor cell population with the FGFR1 inhibitor, the number of cells of interest may be drastically decreased. In the case of treating a population of an early insulin-producing cell or a cell at a later stage of differentiation with the FGFR1 inhibitor, such drastic decrease in the number of cells of interest can be circumvented. Specifically, in the case of treating a population of an early insulin-producing cell or a cell at a later stage of differentiation with the FGFR1 inhibitor, the resulting number of insulin-producing cells can be increased as compared with the case of treating an endocrine progenitor cell population with the FGFR1 inhibitor.

The treatment of an endocrine progenitor cell population or a cell population at a later stage of differentiation with the FGFR1 inhibitor can inhibit the proliferation of Ki67-positive cells in the cell population.

This approach can decrease or suppress the number of remaining proliferative cells contained in the pancreatic lineage, not inhibiting the proliferation of teratoma.

This approach can decrease or suppress the number of remaining proliferative cells contained in the pancreatic lineage, not inhibiting the proliferation of iPS cells (for example, not having to decrease the number of alkaline phosphatase-positive cells).

This approach is capable of reducing the absolute number of Ki67-positive cells in an endocrine progenitor cell population or a cell population at a later stage of differentiation by treatment using an FGFR1 inhibitor. This can deplete Ki67-positive cells in the obtained cell population.

Specifically, the proportion of Ki67-positive cells in the obtained cell population can be reduced as compared with the case of culture and/or differentiation without treatment with the FGFR1 inhibitor. The proportion can be less than 10%, less than 9%, less than 8%, less than 7%, less than 6%, less than 5%, less than 4%, less than 3%, less than 2%, or less than 1% and can be preferably less than 3%, less than 2%, or less than 1%.

According to this approach, the endocrine progenitor cell population or the cell population at a later stage of differentiation treated using the FGFR1 inhibitor can be differentiated into insulin-producing cells or pancreatic β cells to obtain a cell population having inhibited growth of Ki67-positive cells and including enriched insulin-producing cells or pancreatic β cells. Specifically, the proportion of insulin-producing cells or pancreatic β cells in the cell population obtained after induction of differentiation can be increased as compared with a cell population obtained without treatment with the FGFR1 inhibitor. The proportion can be 40% or more, 50% or more, 60% or more, 70% or more, 80% or more, or 90% or more.

The insulin-producing cells or the pancreatic β cells obtained by this approach may be indwelled as they are and used as insulin-secreting cells, when transplanted into a living body of an animal and differentiated in the living body of the animal. The insulin-producing cells or the pancreatic β cells obtained by this approach may circumvent the proliferation of Ki67-positive cells and achieve safe and long-term graft survival of transplanted cells.

According to the present invention, the insulin-producing cell population or the pancreatic β cell population from which highly proliferative Ki67-positive cells have been removed (cell population of the present invention) is transplanted as it is or in a capsule form to an affected area and is thereby useful as a cell medicine for treating diabetes mellitus, particularly, type I diabetes mellitus.

The cell population of the present invention may be a prodrug. The prodrug refers to a cell population that is differentiated after transplantation into a living body and converted to cells having a function of treating a disease.

The cell population of the present invention has low toxicity (for example, acute toxicity, chronic toxicity, genetic toxicity, reproductive toxicity, cardiotoxicity, and carcinogenicity) and can be safely administered as it is or in the form of a pharmaceutical composition containing the cell population mixed with a pharmacologically acceptable carrier, etc. to a mammal (for example, a mouse, a rat, a hamster, a rabbit, a cat, a dog, cattle, sheep, a monkey, and a human).

Hereinafter, the present invention will be described with reference to Examples. However, the present invention is not limited by these Examples.

### Examples

The induction of differentiation of pluripotent stem cells into an endocrine progenitor cell population was carried out according to the above steps 1)-5), the previous report (Stem Cell Research (2015) 14, 185-197), etc.

### Example 1: Decrease in the number of unintended cells (Ki67-positive cells) in cell population obtained by treating endocrine progenitor cell population with FGF receptor 1 inhibitor

### 1. Method

### (1) Preparation of insulin-producing cell population

The induction of differentiation of the endocrine progenitor cell population into an insulin-producing cell population was carried out according to the above step 6) or the previous report (Nature Biotechnology 2014; 32: 1121-1133).

The endocrine progenitor cell population obtained by the induction of differentiation from iPS cells were cultured for 12 days in a medium for induction of differentiation (Improved MEM/1% B27/Penicillin Streptomycin) containing differentiation factors (ALK5 inhibitor II, T3, LDN, γ-secretase inhibitor RO, and ascorbic acid) and an FGF receptor 1 inhibitor (CAS192705-79-6) to obtain an insulin-producing cell population. The number of Ki67-positive cells in the obtained cell population was evaluated by flow cytometry.

### (2) Transplantation of FGF receptor 1 inhibitor-treated cells into living body

In the description below, the preparation of mice and the transplantation of cells were performed in accordance with known methods.

The insulin-producing cell population obtained by culture in the medium for induction of differentiation containing CAS192705-79-6 in the preceding section (1) or an insulin-producing cell population obtained by culture in a medium for induction of differentiation free from CAS192705-79-6 as a comparative example was transplanted beneath the capsule of the kidney using immunodeficient NOD/SCID mice having streptozotocin-induced insulin-deficient diabetes mellitus. For follow-up after the transplantation, human C-peptide concentrations in blood and blood glucose levels were measured. The grafts were excised 5 weeks after the transplantation.

### (3) Evaluation of unintended cells in graft

The excised grafts were fixed and dehydrated, and then, frozen sections were prepared and subjected to immunohistological staining in order to evaluate unintended cells. The cells of interest that could be expected to maturate into pancreatic islet cells *in vivo* were evaluated using insulin positivity (and NKX6.1 positivity) or glucagon positivity as an indicator, and unintended cells having the possibility of adversely affecting long-term graft survival of the cells of interest were evaluated using Ki67 positivity as an indicator.

### 2. Results

### (1) Preparation of insulin-producing cell population

An experiment to treat cells for 12 days with a medium for induction of differentiation containing CAS192705-79-6 (1 µM) was conducted five times. Results about the proportion of Ki67-positive cells ± standard deviation in the obtained cell population are shown in Table 1. In the case of treating an endocrine progenitor cell population with CAS192705-79-6 for 12 days, the proportion of Ki67-positive cells in the obtained cell population was much smaller than that in a control. These results indicate that the treatment of an endocrine progenitor cell population with CAS192705-79-6 decreases the number of Ki67-positive cells or inhibits the proliferation thereof.

**[Table 1]**

| FGF receptor inhibitor treatment | Absent | Present (1 µM) |
|---|---|---|
| Content percentage of Ki67-positive cells (before transplantation) | 10.78 ± 4.09% | 0.98 ± 0.29% |

### (2) Transplantation of FGF receptor 1 inhibitor-treated cells into living body

Results about human C-peptide concentrations in blood and blood glucose levels measured for follow-up after transplantation are shown in Table 2. The insulin-producing cell population obtained by treatment for 12 days with a medium for induction of differentiation containing CAS192705-79-6 (1 µM) secreted human C-peptide into blood and exhibited an effect of improving high blood glucose, 4 months after transplantation. Their levels were equivalent to those of the case of transplanting an insulin-producing cell population obtained without treatment with CAS192705-79-6.

**[Table 2]**

| FGF receptor inhibitor treatment | Absent | Present (1 µM) |
|---|---|---|
| Human C-peptide concentration in blood 4 months after transplantation (pM) | 1440 ± 573 | 1486 ± 305 |
| Blood glucose level at time of transplantation and 4 months after transplantation (mg/dL) | 516 ± 35 (at time of transplantation) | 516 ± 33 (at time of transplantation) |
| | 85 ± 16 (4 months later) | 86 ± 22 (4 months later) |

### (3) Evaluation of unintended cells in graft

Results of immunohistologically staining grafts excised 5 weeks after transplantation are shown in Figure 1.

In the case of transplanting, beneath the capsule of the kidney, an insulin-producing cell population obtained by treatment for 12 days with a medium for induction of differentiation containing CAS192705-79-6 (1 µM), drastic inhibition of bloatedness of the grafts was found as compared with the case of transplanting, beneath the capsule of the kidney, an insulin-producing cell population obtained without treatment with CAS192705-79-6. These results indicate that the treatment of an endocrine progenitor cell population with CAS192705-79-6 decreases the number of Ki67-positive cells, which are unintended cells, or inhibits the proliferation thereof.

### Example 2: Decrease in the number of unintended cells (Ki67-positive cells) and maintenance of cells of interest (insulin-positive (and NKX6.1-positive) cells) in cell population obtained by treating insulin-producing cell population with FGF receptor 1 inhibitor

### 1. Method

1) An endocrine progenitor cell population obtained by the induction of differentiation from iPS cells were cultured for 11 days in a medium for induction of differentiation (Improved MEM/1% B27/Penicillin Streptomycin) containing differentiation factors (ALK5 inhibitor II, T3, LDN, γ-secretase inhibitor RO, and ascorbic acid) and an FGF receptor 1 inhibitor (CAS192705-79-6) to obtain an insulin-producing cell population.
2) An endocrine progenitor cell population obtained by the induction of differentiation from iPS cells were cultured for 4 days in a medium for induction of differentiation (Improved MEM/1% B27/Penicillin Streptomycin) containing differentiation factors (ALK5 inhibitor II, T3, LDN, γ-secretase inhibitor RO, and ascorbic acid) and thereby induced to differentiate into an insulin-producing cell population. Subsequently, an FGF receptor 1 inhibitor (CAS192705-79-6, 1 µM) was added to a medium for induction of differentiation (Improved MEM/1% B27/Penicillin Streptomycin) containing differentiation factors (ALK5 inhibitor II, T3, LDN, γ-secretase inhibitor RO, and ascorbic acid), and the cells were cultured therein for 7 days.
3) An endocrine progenitor cell population obtained by the induction of differentiation from iPS cells were cultured for 7 days in a medium for induction of differentiation (Improved MEM/1% B27/Penicillin Streptomycin) containing differentiation factors (ALK5 inhibitor II, T3, LDN, γ-secretase inhibitor RO, and ascorbic acid) and thereby induced to differentiate into an insulin-producing cell population. Subsequently, an FGF receptor 1 inhibitor (CAS192705-79-6, 1 µM) was added to a medium for induction of differentiation (Improved MEM/1% B27/Penicillin Streptomycin) containing differentiation factors (ALK5 inhibitor II, T3, LDN, γ-secretase inhibitor RO, and ascorbic acid), and the cells were cultured therein for 4 days.
4) An endocrine progenitor cell population obtained by the induction of differentiation from iPS cells were cultured for 11 days in a medium for induction of differentiation (Improved MEM/1% B27/Penicillin Streptomycin) containing differentiation factors (ALK5 inhibitor II, T3, LDN, γ-secretase inhibitor RO, and ascorbic acid) and containing no CAS192705-79-6 and thereby induced to differentiate into an insulin-producing cell population.

The number of Ki67-positive cells in the cell population obtained by each of the above methods 1), 2), and 3) was counted by flow cytometry to determine the proportion of Ki67-positive cells in each cell population.

The number of insulin-positive (and NKX6.1-positive) cells in the cell population obtained by each of the above methods 1), 2), and 3) was counted by flow cytometry. The proportion of insulin-positive (and NKX6.1-positive) cells in each method was calculated as a relative value when the number of insulin-positive (and NKX6.1-positive) cells in the control cell population obtained by the above method 4) was defined as 100%.

### 2. Results

An experiment using each method was repeated three times to evaluate the influence of the timing of treatment with CAS192705-79-6 in an insulin-producing cell production step. Results about mean proportions of Ki67-positive cells and insulin-positive (and NKX6.1-positive) cells obtained in each method ± standard deviation are shown in Table 3.

The proportion of Ki67-positive cells in the obtained cell population did not significantly differ among the case of treating cells using CAS192705-79-6 from the start of the step of induction of differentiation, the case of treating cells for last 7 days, and the case of treating cells for last 4 days, and was much smaller in all the cases than that of the control. These results indicate that treatment with CAS192705-79-6 in the process of producing insulin-producing cells or pancreatic β cells can decrease the number of Ki67-positive cells in the cell population or inhibit the proliferation thereof.

On the other hand, the proportion of insulin-positive (and NKX6.1-positive) cells markedly differed between the case of treating cells from the start of the step of induction of differentiation and the case of treating cells for last 7 days or 4 days. Specifically, in the case of treating cells (endocrine progenitor cell population) with CAS192705-79-6 from the start of the step of induction of differentiation, it was confirmed that the number of insulin-positive (and NKX6.1-positive) cells, which were the cells of interest, was markedly decreased. By contrast, in the case of treating cells (population of early insulin-producing cells or cells at a later stage of differentiation) with CAS192705-79-6 at a stage where differentiation progressed to some extent 4 days or 7 days after the start of induction of differentiation, it was confirmed that the number of insulin-positive (and NKX6.1-positive) cells, which were the cells of interest, was rarely decreased.

**[Table 3]**

| FGF receptor inhibitor treatment | Absent | Present (1 µM) From start | Present (1 µM) For last 7 days | Present (1 µM) For last 4 days |
|---|---|---|---|---|
| Proportion of Ki67-positive cells | 7.57 ± 1.05% | 0.57 ± 0.35% | 0.47 ± 0.15% | 0.57 ± 0.15% |
| Proportion of insulin-positive (and NKX6.1-positive) cells | 100 ± 18% | 45 ± 15% | 88 ± 13% | 91 ± 16% |

### Example 3: Decrease in the number of unintended cells (Ki67-positive cells) in cell population obtained by treatment with FGFR1 inhibitor other than CAS192705-79-6 1. Method

An endocrine progenitor cell population obtained by the induction of differentiation from iPS cells were cultured for 8 days in a medium for induction of differentiation (Improved MEM/1% B27/Penicillin Streptomycin) containing differentiation factors (ALK5 inhibitor II, T3, LDN, γ-secretase inhibitor RO, and ascorbic acid). Subsequently, CAS192705-79-6, E-3810, or PD173074 was added at three concentrations (low, medium, and high) to a medium for induction of differentiation (MCDB131/20 mM Glucose/NaHCO₃/FAF-BSA/ITS-X/Glutamax/ascorbic acid/Penicillin Streptomycin) containing differentiation factors (T3, ALK5 inhibitor II, ZnSO₄, heparin, N-acetylcysteine, Trolox, and R428), and the cells were cultured in the medium for 4 days. The number of Ki67-positive cells in the obtained cell population was evaluated by flow cytometry.

### 2. Results

The proportion of Ki67-positive cells in a cell population obtained by treatment with CAS192705-79-6 or E-3810 is shown in Table 4. Also, the proportion of Ki67-positive cells in a cell population obtained by treatment with PD173074 is shown in Table 5.

From these results, it was confirmed that in cell populations obtained by treatment not only with CAS192705-79-6 but with other FGFR1 inhibitors, the proportion of Ki67-positive cells was much lower than that of the control. These results indicate that the inhibition treatment of FGFR1 without being limited by CAS192705-79-6 treatment decreases the number of Ki67-positive cells or inhibits the proliferation thereof.

**[Table 4]**

| FGFR1 inhibitor | Proportion of Ki67-positive cells (%) ; Treatment concentration (µM) | | |
|---|---|---|---|
| Absent | 4.13% | - | - |
| CAS192705-79-6 | 2.15% ; | 0.99% ; | 0.7%; |
| | 0.1 µM | 0.3 µM | 1 µM |
| E-3810 | 2.74%; | 1.51%; | 0.67% ; |
| | 0.3 µM | 1 µM | 10 µM |

**[Table 5]**

| FGFR1 inhibitor | Proportion of Ki67-positive cells (%) ; Treatment concentration (µM) | | |
|---|---|---|---|
| Absent | 2.7% | - | - |
| PD-173074 | 0.7%; | 0.7%; | 0.5%; |
| | 0.1 µM | 1 µM | 5 µM |

The results described above demonstrated that the treatment of a population of endocrine progenitor cells or cells at a later stage of differentiation with an FGFR1 inhibitor is capable of decreasing the number of Ki67-positive cells present in the cell population or inhibiting the proliferation thereof, and as a result, a cell population enriched in the cells of interest can be obtained.

## Claims

1. A method for producing an insulin-producing cell population or a pancreatic β cell population, comprising the step of
treating an insulin-producing cell population or a pancreatic β cell population with an FGFR1 inhibitor.

2. The method according to claim 1, further comprising the step of differentiating the insulin-producing cell population treated with the FGFR1 inhibitor.

3. The method according to claim 1 or 2, wherein the insulin-producing cell population or the pancreatic β cell population is treated with less than 5 µM of the FGFR1 inhibitor.

4. The method according to any one of claims 1 to 3, wherein the produced cell population comprises Ki67-positive cells at a proportion of less than 3%.

5. The method according to any one of claims 1 to 4, wherein the FGFR1 inhibitor is 1-[2-amino-6-(3,5-dimethoxyphenyl)-pyrido(2,3-d)pyrimidin-7-yl]-3-tert-butylurea or a salt thereof.

6. The method according to any one of claims 2 to 5, wherein the step of differentiating the insulin-producing cell population treated with the FGFR1 inhibitor is performed by transplantation to an animal.

7. A method for producing an insulin-producing cell population or a pancreatic β cell population, comprising the step of
treating a population of endocrine progenitor cells or cells at a later stage of differentiation with less than 5 µM of an FGFR1 inhibitor.

8. A method for decreasing the number of or inhibiting the proliferation of Ki67-positive cells present in a population of endocrine progenitor cells or cells at a later stage of differentiation, comprising
treating the cell population with an FGFR1 inhibitor.

9. The method according to claim 8, wherein the population of endocrine progenitor cells or cells at a later stage of differentiation is an insulin-producing cell population.

10. The method according to claim 8 or 9, wherein the population of endocrine progenitor cells or cells at a later stage of differentiation is treated with less than 5 µM of the FGFR1 inhibitor.

11. The method according to any one of claims 8 to 10, wherein the FGFR1 inhibitor is 1-[2-amino-6-(3,5-dimethoxyphenyl)-pyrido(2,3-d)pyrimidin-7-yl]-3-tert-butylurea or a salt thereof.

12. A population of pancreatic progenitor cells or cells at a later stage of differentiation, comprising Ki67-positive cells at a proportion of less than 3%.

13. The cell population according to claim 12, wherein the cell population is an insulin-producing cell population.

14. The cell population according to claim 12 or 13, wherein the cell population is used for transplantation.
